(19) Europäisches Patentamt
European Patent Office
Office européen des brevets

(11) EP 3 034 624 A1

(12) EUROPEAN PATENT APPLICATION

(43) Date of publication:
**22.06.2016 Bulletin 2016/25**

(51) Int Cl.:
***C12Q 1/68*** *(2006.01)*

(21) Application number: **14382535.4**

(22) Date of filing: **18.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Hospital Clínic de Barcelona**
  **08036 Barcelona (ES)**
- **Institut d'Investigacions Biomédiques August Pi i Sunyer**
  **08036 Barcelona (ES)**
- **Fundació Institut d'Investigació Biomèdica de Bellvitge (IDIBELL)**
  **08907 L'Hospitalet de Llobregat (ES)**
- **Centro de Investigación Biomédica en Red (CIBER)**
  **28029 Madrid (ES)**
- **Institució Catalana de Recerca i Estudis Avançats**
  **08010 Barcelona (ES)**

(72) Inventors:
- **Villanueva Rodriguez, Augusto**
  **08036 Barcelona (ES)**
- **Llovet Bayer, Josep Maria**
  **08036 Barcelona (ES)**
- **Esteller Badosa, Manel**
  **08908 L'Hospitalet del Llobregat (ES)**
- **Portela Mestres, Anna**
  **08908 L'Hospitalet del Llobregat (ES)**
- **Sayols Puig, Sergi**
  **08908 L'Hospitalet del Llobregat (ES)**

(74) Representative: **ZBM Patents - Zea, Barlocci & Markvardsen**
**Plaza Catalunya, 1**
**08002 Barcelona (ES)**

# (54) Method for the prognosis of hepatocellular carcinoma

(57) The invention relates to a method for the prognosis of hepatocellular carcinoma in an isolated sample of a subject, the method comprising providing a DNA methylation signature, said signature obtained from the qualitative and quantitative methylation analysis of 36 selected CpG sites. The invention also discloses particular methods in which a Mortality Index score is provided from said signature.



Printed by Jouve, 75001 PARIS (FR)

SEQUENCE LISTING

<110> Hospital Clínic de Barcelona
Institut d'Investigacions Biomediques August Pi i Sunyer (IDIBAPS)
Fundacio Institut d'Investigacio Biomedica de Bellvitge (IDIBELL)
Centro de Investigacion Biomodica en Red (CIBER)
INSTITUCIO CATALANA DE RECERCA I ESTUDIS AVANCATS (ICREA)

<120> Method for the prognosis of hepatocellular carcinoma

<130> P3156EP00

<160> 36

<170> PatentIn version 3.5

<210> 1
<211> 122
<212> DNA
<213> Homo sapiens

<220>
<221> misc_feature
<222> (61)..(61)
<223> CpG site in chromosome 20, cytosine at position 25062754: cg00043788 reference of Illumina®'s CpG Loci Identification

<400> 1
gctagtgcgc ccgtcggaaa gcgagtcccg gccggtcatg gttccttagc aagcaaggcg 60

cgagcctctc tggatcccgt ttgcggaggg cccagcttag aggaagcttt ataggattgg 120

gc 122

<210> 2
<211> 122
<212> DNA
<213> Homo sapiens

<220>
<221> misc_feature
<222> (61)..(61)
<223> CpG site in chromosome 16, cytosine at position 67571354: cg00586537 reference of Illumina®'s CpG Loci Identification

<400> 2
cggtcccgcc ccatccaggc gggctgagtc aggcggcagg aactggcgg ggggcggcgc 60

cgggaggagc cgaagccgag ccagagccgc tgggagcgag cccggagccc agccgggcgg 120

ct 122

<210> 3
<211> 122
<212> DNA
<213> Homo sapiens

<220>
<221> misc_feature
<222> (61)..(61)
<223> CpG site of chromosome 6, cytosine at position 112194776: cg00696540 reference of Illumina®'s CpG Loci Identification

<400> 3
ccggctgccc tatcaaagag ccccttggga gctgcgagcc gcaaccattg gcgcctgggg 60

cgtgggcggg acggcagtag gtggggccgc cgagtgggtt gagcgttact gtggcagcgg 120

gc 122

<210> 4
<211> 122
<212> DNA
<213> Homo sapiens

<220>
<221> misc_feature
<222> (61)..(61)
<223> CpG site of chromosome 3, cytosine at position 147127012: cg01227537 reference of Illumina®'s CpG Loci Identification

<400> 4
gatgtcaagc gctttacaat acctgggatt gatgaggcgg gcgggccaat gagctgcgcg 60

cggcgcctcg gcgcgccctc cgttggcgcg gcggctgagg gcggggggaa tgcgggcgca 120

cc 122

<210> 5
<211> 122
<212> DNA
<213> Homo sapiens

<220>
<221> misc_feature
<222> (61)..(61)
<223> CpG site of chromosome 2, cytosine at position 47797415: cg02318629 reference of Illumina®'s CpG Loci Identification

<400> 5
aagcggccgg tgtcctcgtt gaggtgcgaa cggcggcagc agcagcagca gcaggagggg 60

cgcggcaggc ggcggcggct acggcggggc gggggccggg ggctgcggga ggacatggtc 120

cg 122

<210> 6
<211> 122
<212> DNA
<213> Homo sapiens

<220>
<221> misc_feature

<222> (61)..(61)
<223> CpG site of chromosome 19, cytosine at position 38747378: cg02571816 reference of Illumina®'s CpG Loci Identification

<400> 6
gcggggtggg gggctgttgg tgcgggagcg cctgcgccgc tgtcgctgta gaaggtcgag 60

cggtcacttg ttccttcgtt cattcattca ctcatccatc catttgtttg ttcactcatt 120

cc 122

<210> 7
<211> 122
<212> DNA
<213> Homo sapiens

<220>
<221> misc_feature
<222> (61)..(61)
<223> CpG site of chromosome 8, cytosine at position 94928855: cg03578886 reference of Illumina®'s CpG Loci Identification

<400> 7
acattggagc cggcccaggc gcctcccctc tcccgacccc gcagcctgct cagccaacac 60

cgccgagctc cacagaccac cgctccctag cgccgcgcag ccacaccttc cactcgctgg 120

gg 122

<210> 8
<211> 122
<212> DNA
<213> Homo sapiens

<220>
<221> misc_feature
<222> (61)..(61)
<223> CpG site of chromosome 21, cytosine at position 34443010: cg03732762 reference of Illumina®'s CpG Loci Identification

<400> 8
tgccctactc agcggcgcac tgccagggcg cgcccggccg caagctctcc aagatagcca 60

cgctgctgct cgcccgcaac tacatcctac tgctgggcag ctcgctgcag gagctgcgcc 120

gc 122

<210> 9
<211> 122
<212> DNA
<213> Homo sapiens

<220>
<221> misc_feature
<222> (61)..(61)
<223> CpG site of chromosome 16, cytosine at position 30022699: cg03920233 reference of Illumina®'s CpG Loci Identification

<400> 9
tggcacactt acccggagaa cttcgcacac acacgcactc gcaaacacgc gggctccctg 60

cgccaccagg aagcagccgc cagaatcgcc gggtgttccc caactactcc tgggagacct 120

gg 122

<210> 10
<211> 122
<212> DNA
<213> Homo sapiens

<220>
<221> misc_feature
<222> (61)..(61)
<223> CpG site of chromosome 14, cytosine at position 24899054: cg04274978 reference of Illumina®'s CpG Loci Identification

<400> 10
ggagggctgg tgagaagcgc aaggggcgag ccctggagcc gccgagggga ctaggccgag 60

cgggcccgtc ggggcctctg ggcgcggcgg cggggttggg aggagggcgc gcagccggga 120

gg 122

<210> 11
<211> 122
<212> DNA
<213> Homo sapiens

<220>
<221> misc_feature
<222> (61)..(61)
<223> CpG site of chromosome 13, cytosine at position 107188416: cg04321866 reference of Illumina®'s CpG Loci Identification

<400> 11
cccctcggcg gaatcagagg gcgcggcttt ccctcgagca gtgcggaagg cagcaggctc 60

cgctcggcgt cccttctccg cagatgcggc gctgccccgc gtgcaagact tgcactccgc 120

gc 122

<210> 12
<211> 122
<212> DNA
<213> Homo sapiens

<220>
<221> misc_feature
<222> (61)..(61)
<223> CpG site of chromosome 4, cytosine at position 48908469: cg04961553 reference of Illumina®'s CpG Loci Identification

<400> 12
acgggggaca ctgcactttc tgatgcggtc ggggaagccg ggagtcagcc tactggagtc 60

cgctgccatc cctggccaga agcagccagc gccaccccaa gctccccagc ccctcgcccg 120

tg 122

<210> 13
<211> 122
<212> DNA
<213> Homo sapiens

<220>
<221> misc_feature
<222> (61)..(61)
<223> CpG site of chromosome 7, cytosine at position 108096745: cg05412664 reference of Illumina®'s CpG Loci Identification

<400> 13
acgttctcgc gccgcgccct ccgctcagct ctggcgcgac tgcccaggac cctggaccgc 60

cggtgtcctc cgttctcgac gaagctatcc cctctggctg ctccagccag gcggcccctg 120

cg 122

<210> 14
<211> 122
<212> DNA
<213> Homo sapiens

<220>
<221> misc_feature
<222> (61)..(61)
<223> CpG site of chromosome 10, cytosine at position 75671378: cg06521280 reference of Illumina®'s CpG Loci Identification

<400> 14
gagccctgct ggcgcgcctg cttctctgcg tcctggtcgt gagcgactcc aaagtgagtg 60

cgctcttgct ttgactgatg ctgcccaagg acctctgatc agcaccaggg gagaggaggg 120

gc 122

<210> 15
<211> 122
<212> DNA
<213> Homo sapiens

<220>
<221> misc_feature
<222> (61)..(61)
<223> CpG site of chromosome 8, cytosine at position 8559999: cg06671706 reference of Illumina®'s CpG Loci Identification

<400> 15
atggtgttgg cgccctgcgg gctcctgctc aacctgaccg gcaccctggc gcccggctgg 60

cggctggtga agggcttcct gaaccagcca gtggacgtgg agttgtacca gggcctgtgg 120

ga 122

<210> 16
<211> 122
<212> DNA
<213> Homo sapiens

<220>
<221> misc_feature
<222> (61)..(61)
<223> CpG site of chromosome 7, cytosine at position 95401692: cg07732116 reference of Illumina®'s CpG Loci Identification

<400> 16
gcaggagctc ccagaatatt gaagctcttc tctttgcagc aactgaggtc taagtcctac 60

cgacgttaga gggatagacg aattgacctt tccaggtcct ctgctcgcca ccagcctacg 120

ga 122

<210> 17
<211> 122
<212> DNA
<213> Homo sapiens

<220>
<221> misc_feature
<222> (61)..(61)
<223> CpG site of chromosome 10, cytosine at position 97803058: cg08379212 reference of Illumina®'s CpG Loci Identification

<400> 17
tggcctaact ggattggtca ctgcagtgat ccaccttaca atttctccgg cgctctctgc 60

cgacctcagc cggcgatcag gccggcttcg cagtaggtgg cggctgagcg ccccggcaac 120

cg 122

<210> 18
<211> 122
<212> DNA
<213> Homo sapiens

<220>
<221> misc_feature
<222> (61)..(61)
<223> CpG site of chromosome 14, cytosine at position 23821445: cg09321747 reference of Illumina®'s CpG Loci Identification

<400> 18
cagcgggggc gccagcgtga agatggggtc cgaggccatg cccagagcca cgaagagcac 60

cggcaggcag cagaggccga gctgcagctg ctggccgccg cccagcgccc ccacctgggc 120

ga 122

<210> 19
<211> 122
<212> DNA
<213> Homo sapiens

<220>
<221> misc_feature
<222> (61)..(61)
<223> CpG site of chromosome 15, cytosine at position 83378614: cg09472203 reference of Illumina®'s CpG Loci Identification

<400> 19
gcgaaggcgg cggcgcggca ggggttcagc agaggctgca gtgtgcagcg gcggaggctg 60

cgcgcggatt tctcaatcag ggccgcgcgc tgaggtctta aaggcaccgg cgccagccga 120

gc 122

<210> 20
<211> 122
<212> DNA
<213> Homo sapiens

<220>
<221> misc_feature
<222> (61)..(61)
<223> CpG site of chromosome 7, cytosine at position 155249746: cg10481660 reference of Illumina®'s CpG Loci Identification

<400> 20
ataagtgtct gcaggaggag tgtcctgcgc gccagctctg cgtttaagac aggaagctgc 60

cgggttaccg agtcaaatgg gagtgacact attcctctcc atcagcaagg aaagcggacc 120

ac 122

<210> 21
<211> 122
<212> DNA
<213> Homo sapiens

<220>
<221> misc_feature
<222> (61)..(61)
<223> CpG site of chromosome 18, cytosine at position 24130633: cg12881557 reference of Illumina®'s CpG Loci Identification

<400> 21
gctctcatcc gcccggctct ccgcggagaa ccgtccggga atcgtccccg aggtgcggcg 60

cgggaggtgg ccggctttgt tcacaatgca ctgcccgccg cacccgccct ggacacgtgc 120

ag 122

<210> 22
<211> 122
<212> DNA

<213> Homo sapiens

<220>
<221> misc_feature
<222> (61)..(61)
<223> CpG site of chromosome 2, cytosine at position 26624823: cg13414212 reference of Illumina®'s CpG Loci Identification

<400> 22
tgaccgctct ccctggcaac ggtttgttcc tagcaaccag cctgaggtct ggaggtggtg 60

cggagggagc cgcctaggga ccagggactc ctgccatgaa tccgccgggg tccctagagg 120

cc 122

<210> 23
<211> 122
<212> DNA
<213> Homo sapiens

<220>
<221> misc_feature
<222> (61)..(61)
<223> CpG site of chromosome 2, cytosine at position 45236267: cg15019790 reference of Illumina®'s CpG Loci Identification

<400> 23
ccacttgctc ctgcgtgaag ccgaaggtgg gcagcatgga catggtgccg gctgcgtccc 60

cgcccgcccg cgcgcgccct caccgggccg cgcggtcccg catgggagct tcctcgccgg 120

gc 122

<210> 24
<211> 122
<212> DNA
<213> Homo sapiens

<220>
<221> misc_feature
<222> (61)..(61)
<223> CpG site of chromosome 14, cytosine at position 69726620: cg15065139 reference of Illumina®'s CpG Loci Identification

<400> 24
tgccgggcgt tcggtcacct agcggtggga gccgaggctg gctttggcca gtcgccattc 60

cgccgcgggc gtctgtctcg ccgggttccc gggcgcggga ggaaggcccg cgccgcttcc 120

ca 122

<210> 25
<211> 122
<212> DNA
<213> Homo sapiens

<220>
<221> misc_feature
<222> (61)..(61)
<223> CpG site of chromosome 5, cytosine at position 114632040: cg16113530 reference of Illumina®'s CpG Loci Identification

<400> 25
agagcaacgc ccagcgcgtg ccaggccccg agcaggggag gcgaacgccg cgcctcccgc 60

cggcgctgca gagggcacct gttcagccag ggcctgcagc ccctcgcctg ccgccagagc 120

ag 122

<210> 26
<211> 122
<212> DNA
<213> Homo sapiens

<220>
<221> misc_feature
<222> (61)..(61)
<223> CpG site of chromosome 2, cytosine at position 74648928: cg17157630 reference of Illumina®'s CpG Loci Identification

<400> 26
cgtcgtctct atggtggcgg cggatttgga gggaccctac gaaccaggag tcaggcgagc 60

cgatctgggg ctgcaggtgt tacctctgat ctaggccggg ggcttcaggg atccgagccg 120

ag 122

<210> 27
<211> 122
<212> DNA
<213> Homo sapiens

<220>
<221> misc_feature
<222> (61)..(61)
<223> CpG site of chromosome 17, cytosine at position 66193820: cg17179589 reference of Illumina®'s CpG Loci Identification

<400> 27
ggggaagggg cgccggggca gtgtcctcct tggggtttgt tggtccagct ctgggcagct 60

cgatgacccc gcctccggga cccagtgtgc ttagtgactc agtttacact ttttcctcgt 120

cg 122

<210> 28
<211> 122
<212> DNA
<213> Homo sapiens

<220>
<221> misc_feature
<222> (61)..(61)

<223> CpG site of chromosome 3, cytosine at position 159943073: cg18703913 reference of Illumina®'s CpG Loci Identification

<400> 28
agagcgttaa cccttccagt cccagagagc gacaaggcgg gggaggaaaa acgcgccggc 60

cggggccaag atgcccatgg cagccccgcg cgggctgcct ctgacattta gggaggctcc 120

gg 122

<210> 29
<211> 122
<212> DNA
<213> Homo sapiens

<220>
<221> misc_feature
<222> (61)..(61)
<223> CpG sit eof chromosome 16, cytosine at position 2932904: cg21085535 reference of Illumina®'s CpG Loci Identification

<400> 29
gtcaaggatg cagagcttaa agcaaggcag tggcttccat gagcctaaac accttccctc 60

cgcacgctcg gtatcgcgac gggggagagc aaaaccttta aacaaatgct ccagttttcc 120

cc 122

<210> 30
<211> 122
<212> DNA
<213> Homo sapiens

<220>
<221> misc_feature
<222> (61)..(61)
<223> CpG site of chromosome 7, cytosine at position 5633086: cg22447539 reference of Illumina®'s CpG Loci Identification

<400> 30
gcacctgagc gcgcggccgg ccgacgagat cgccgtggac cgcgacgtgc cctggggcgt 60

cgactcgctc atcaccctcg ccttccagga ccagcgctac agcgtgcaga ccgccgacca 120

cc 122

<210> 31
<211> 122
<212> DNA
<213> Homo sapiens

<220>
<221> misc_feature
<222> (61)..(61)
<223> CpG site of chromosome 17, cytosine at position 21279621: cg22639895 reference of Illumina®'s CpG Loci Identification

<400> 31
cgggactagg aggggctcgg ggccaggccg gagccgtggt aggctggtgc agcggctgcg 60

cggcgcggcg cggcgcgggg cgggcgtgct ctccgcggag agttagagga gttgccgagc 120

tg 122

<210> 32
<211> 122
<212> DNA
<213> Homo sapiens

<220>
<221> misc_feature
<222> (61)..(61)
<223> CpG site of chromosome 11, cytosine at position 35639656: cg23083315 reference of Illumina®'s CpG Loci Identification

<400> 32
gaggtggaaa tctgtctgaa gtttgcagga gacccgcgct gcctaggcaa agggacccgg 60

cgaccaagac acccccgcga gggcccaggg cgcgtccctc ccgccaggcc ccagactccg 120

gc 122

<210> 33
<211> 122
<212> DNA
<213> Homo sapiens

<220>
<221> misc_feature
<222> (61)..(61)
<223> CpG site of chromosome 18, cytosine at position 32847789: cg23624808 reference of Illumina®'s CpG Loci Identification

<400> 33
attcaaacat atttttcctc gttatgtcgc gggctggtat accatcgaga aaataaaaac 60

cgagaaccca tgatctctac attttgagag gaagaggagc tgaccacgct caccgcggac 120

gt 122

<210> 34
<211> 122
<212> DNA
<213> Homo sapiens

<220>
<221> misc_feature
<222> (61)..(61)
<223> CpG site of chromosome 3, cytosine at position 140660432: cg23664775 reference of Illumina®'s CpG Loci Identification

<400> 34
ccgccagttc cctgttcagg agagtgcagc cgtgattggc gcgttcactc aggccagggg 60

```
cgccccaggt ctcaggtgct aggagcgcgg tgacctacca ccctccgggc acgcgttctg      120

gc                                                                      122


<210>  35
<211>  122
<212>  DNA
<213>  Homo sapiens


<220>
<221>  misc_feature
<222>  (61)..(61)
<223>  CpG site of chromosome 17, cytosine at position 79197398:
       cg26527638 reference of Illumina®'s CpG Loci Identification

<400>  35
tccctgacct ccttcccttc gcgctgcttt gttgtttttt ttccttctcc aaagttttaa       60

cgctgcgccc cgtgcgtagc cgctctgtga ctccccctct ctccgggagc cctggggatt      120

cc                                                                      122


<210>  36
<211>  122
<212>  DNA
<213>  Homo sapiens


<220>
<221>  misc_feature
<222>  (61)..(61)
<223>  CpG site of chromosome 16, cytosine at position 67313014:
       cg26793648 reference of Illumina®'s CpG Loci Identification

<400>  36
tacacagaag gccttcccct cccgtcggct cattcagccg cggaagcccc tgggagaccc       60

cgccccgccc cgtcccgcgg tgccccccgc gcactcaccc tcgctcaccc acgcggcctt      120

cc                                                                      122
```

## Description

The present invention relates to the field of medicine, in particular in the field of cancer detection and establishment of a prognosis. It provides methods for the prognosis of cancers, namely hepatocellular cancers.

BACKGROUND ART

Liver cancer represents a major health problem being the second cause of cancer death worldwide. Hepatocellular carcinoma (HCC, also called malignant hepatoma) is the most common type of liver cancer. Most cases of HCC are secondary to either a viral hepatitis infection (hepatitis B or C) or cirrhosis (alcoholism being the most common cause of hepatic cirrhosis). Surgical resection, liver transplantation and local ablation are the recommended treatment options for early hepatocellular carcinoma, but can only be applied to around 30 % of patients in the West. Current clinical practice guidelines recommend resection in patients with single tumors and well-preserved liver function. Even in these cases, tumor recurrence occurs in up to 70 % of patients at 5 years, and no adjuvant therapy is available. Thus, usual outcome or prognosis is poor, because only 10-20% of hepatocellular carcinomas can be removed completely using surgery. If the cancer cannot be completely removed, the disease is usually deadly within 3 to 6 months. The prognosis for metastatic or unresectable hepatocellular carcinoma has recently improved due to the approval of sorafenib (Nexavar®) for advanced hepatocellular carcinoma.

There have been proposed some markers for the prognosis and probability of recurrence of the disease. Among these, there is the document of Villanueva et al. "Combining Clinical, Pathology, and Gene Expression Data to Predict Recurrence of Hepatocellular Carcinoma", Gastroenterolog-2011, Vol. No.140, pp.:1501-2. Villanueva et al. demonstrate how mRNA transcription (mRNA-based gene signatures) from HCC resection specimens and biopsies may improve prognostic performance of conventional clinical and pathological variables.

Similarly, patent application publication US2014004521 (Chao-Ting et al.) discloses that the expression levels of certain micro RNAs (miRNAs), in non-cancerous liver tissue of a subject, are positively or negatively associated with the clinical outcome of the subject. The document mentions as biomarkers miR-486-3p, mir-876-5p, mir-381, mir-30c, mir-432, and mir-15b. Also the patent application publication US2012329672 (Carlo M et al.) discloses a method for identifying a human with poor survival prognosis for HCC, in which levels of other several miRNA are determined.

DNA methylation regulates cell differentiation and participates in tumorogenesis. Global loss of DNA methylation is a hallmark of human cancer, also characterized by selective hypermethylation confined to gene promoters. In HCC, there is no clear understanding of the methylome and epidrivers, and few studies have comprehensively evaluated methylation biomarkers using high-throughput platforms.

A methylation profile associated with HCC risk factor, tumor progression and with prediction of survival is disclosed by Hernández-Vargas et al., "Hepatocellular Carcinoma Displays Distinct DNA Methylation Signatures with Potential as Clinical Predictors", PlosOne 2010 5(3) e9749. These authors propose a particular DNA-methylation signature determined by bead array in 27000 methylation points between HCC tumor samples and normal surrounding tissue. Patients were scored and classified into two groups with significantly different survival curves. Although these represent interesting data, analysed samples derived from a small cohort of patients (n=40) and few is explained on how the DNA-methylation signature could be applied in clinics. Additionally, the test did not include any validation cohort.

On the other hand, methylation signature in HCC using more methylation points was disclosed by Song et al. "Elucidating the Landscape of Aberrant DNA Methylation in Hepatocellular Carcinoma", PlosOne 2013 8 e55761. Song et al. characterized differential methylome (DNA-methylation signature) from two patient samples using the Illumina HumanMethylation 450 Bead Chip kit. From the analysis they concluded that global hypomethylation was observed in HCC, concentrating in the intergenic regions and gene tested bodies. In addition, there were discovered potentially new loci for HCC with a higher density in promoter shores than in CpG islands; they also determined that there was a higher frequency of hypermethylation events in the promoter CpG islands than in the shores and shelves; and that it prevailed an enrichment of promoter CpG island differential methylation loci in gene networks of Cellular development, gene expression, cell death and cancer. The findings may help to better understand epigenetic changes and they were postulated as possible markers for diagnosis, treatment and prognosis of the disease, albeit Song et al. didn't propose any concluding data for any particular methylome (DNA-methylation signature).

Particular methylomes have also been associated with the prognosis of the disease in a subtype of patient. In such a way, the document of Binkui et al, "CpG island Methylator Phenotype Associated with Tumor Recurrence in Tumor-Node-Metastasis Stage I Hepatocellular Carcinoma", Annals of surgical oncology - 2010), Vol. No. 17(7), pp.:1917-26, proposes methylation as a mode for defining a subgroup of patients with poor outcome after resection of tumor. Methylation of particular genes was determined by nested methylation-specific polymerase chain reaction (MSP) in 115 HCC subjects who underwent curative resection. The "methylator phenotype" is proposed as a useful tool for stratifying prognosis of patients and for identifying a higher risk of recurrence.

Wu et al., "Predictive value of CpG island methylator phenotype for tumor recurrence in hepatitis B virus-associated hepatocellular carcinoma following liver transplantation", BMC cancer -2010, Vol. No. 10, pp.: 399, disclose also a particular DNA-methylation signature for the prognosis of recurrence in the subtype of patients with the cancer disease caused or stemed by an hepatitis B virus. Methylome associated with a poor prognosis or with a late-satge of the disease is disclosed by Cheng et al., "Correlation of CpG island methylator phenotype with poor prognosis in hepatocellular carcinoma", Experimental and Molecular Pathology-2010, Vol. No. 88(1), pp.: 112-117.

These particular methylomes for a subset of patients imply precisely the drawback of not being useful for all HCC patient types and, thus they make difficult a real clinical practice.

Albeit several efforts have been made to establish useful markers for the prognosis of HCC, none has been duly translated to the clinical practice. Thus, there is still a need of new approaches that can be used in clinics.

SUMMARY OF THE INVENTION

The inventors propose a new and accurate test for the analysis of the prognosis of HCC diagnosed patients with the aim of improving clinical decisions.

It is proposed determining the methylation profile (or DNA methylation signature) of some genomic regions that inventors found differentially methylated between HCC patients with a good overcome for the disease and patients with a bad prognosis for the disease. Thus, the invention is framed in the field of the use of epigenetics, and more particularly of the DNA methylation profile, signature or pattern of some genomic regions, said regions including genes or surrounding gene areas, namely the promoters of the genes. From this DNA methylation signature a robust parameter can be calculated to aid clinicians in deciding the way to treat HCC in a particular patient.

In a first aspect the invention relates to methods for the prognosis of hepatocellular carcinoma in an isolated sample of a subject, the methods comprising providing a DNA methylation signature, defined by the qualitative and quantitative methylation analysis of a set of cytosines of CpG sites, said set comprising:

Cytosine at position 25062754 of human chromosome 20;
Cytosines at position 67571354, 30022699, 2932904 and 67313014 of human chromosome 16;
Cytosine at position 112194776 of human chromosome 6;
Cytosines at positions 147127012, 159943073 and 140660432 of human chromosome 3;
Cytosines at position 47797415, 26624823, 45236267 and 74648928 of human chromosome 2;
Cytosine at position 38747378 of human chromosome 19;
Cytosines at position 94928855 and 8559999 of human chromosome 8; Cytosine at position 34443010 of human chromosome 21;
Cytosines at position 24899054, 23821445, and 69726620 of human chromosome 14;
Cytosine at position 107188416 of human chromosome 13;
Cytosine at position 48908469 of human chromosome 4;
Cytosines at position 108096745, 95401692, 155249746 and 5633086 of human chromosome 7;
Cytosines at position 75671378 and 97803058 of human chromosome 10; Cytosine at position 83378614 of human chromosome 15;
Cytosines at position 24130633 and 32847789 of human chromosome 18; Cytosine at position 114632040 of human chromosome 5;
Cytosines at position 66193820, 21279621 and 79197398 of human chromosome 17; and
Cytosine at position 35639656 of human chromosome 11;

being all cytosine positions in human chromosomes according to the chromosome map and sequence entries of database UCSC Genome Browser on Human February 2009, GRCh37/hg19 assembly of the University of California Santa Cruz (UCSC).

Chromosome sequences may also be retrieved from the following GenBank database entries, according to the human genome GRCh38 reference primary assembly of December 20, 2013 (versions of sequences are also indicated):

Human chromosome 20 with GenBank accession number CM000682, version 2; human chromosome 16 with GenBank accession number CM000678,version 2;
human chromosome 6 with GenBank accession number CM000668, version 2;
human chromosome 3 with GenBank accession number CM000665, version 2;
human chromosome 2 with GenBank accession number CM000664, version 2;
human chromosome 19 with GenBank accession number CM000681, version 2;
human chromosome 8, with GenBank accession number CM000670, version 2;
human chromosome 21, with GenBank accession number CM000683, version 2;
human chromosome 14, with GenBank accession number CM000676, version 2;

human chromosome 13, with GenBank accession number CM000675, version 2;
human chromosome 4, with GenBank accession number CM000666, version 2;
human chromosome 7, with GenBank accession number CM000669, version 2;
human chromosome 10, with GenBank accession number CM000672, version 2;
human chromosome 15, with GenBank accession number CM000677, version 2;
human chromosome 18, with GenBank accession number CM000680, version 2;
human chromosome 5, with GenBank accession number CM000667, version 2;
human chromosome 17, with GenBank accession number CM000679, version 2; and
human chromosome 11, with GenBank accession number CM000673, version 2.

The method is performed thus by the qualitatively and quantitatively analysis of a set of 36 cytosines of CpG sites. CpG sites are genomic regions of DNA where a cytosine nucleotide occurs next to a guanine nucleotide. For the qualitative and quantitative methylation analysis is to be understood the analysis of the methylation presence or absence of in a cytosine, and of the levels of methylation, if present.

All these 36 cytosines (included in identified CpG sites) can also be defined precisely by identification of their corresponding CpG loci identifier, edited by Illumina Inc (USA), and disclosed in CpG Loci Identification of Pub. No. 270-2007-006 current as of 1 February 2008 (retrievable from http://res.illumina.com/documents/products/technotes/technote_cpg_loci_ident ification.pdf). Table A below correlates Illumina®'s CpG identification with the cytosine in the corresponding chromosome above exposed.

Therefore, by means of the method of the invention the level (quantitative) of DNA methylation, as well as the presence or absence of this methylation (qualitative analysis) is determined from at least these cytosines sited in CpG sites. A DNA-methylation signature specific for each subject can thus be obtained, which can be further confronted with a reference.

Determining the presence or absence of methylation and the methylation level in all of these 36 cytosines gives rise to a precise and highly sensitive prognosis method. Noteworthy, the proposed method and DNA methylation signature of the invention are useful in the prognosis of HCC despite remarkable etiology differences of the samples to be tested. Therefore, the methods of the invention are universal methods indistinctly applicable to C or B hepatitis-related HCC samples as well as to alcohol-related (cirrhosis) HCC samples, as will be illustrated in the examples below. Thus, inventors surprisingly found the group of cytosines allowing classification of subjects suffering from HCC according to its prognosis (outcome prediction), independently of the cancer cause.

The method allows good classification of the subjects suffering from HCC and, at the same time, information of other several aspects of this cancer are elucidated with the method. Some of these additional aspects of the cancer include, among others, recurrence probability after resection of the tumor and the characterization of a subtype of cancer with a particular cytological phenotype.

As above exposed, DNA methylation signatures can be analysed using standardised methodologies, including among others Illumina® HumanMethylation 450 Bead Chip kit. Illumina® HumanMethylation 450 Bead Chip kit is a method based on highly multiplexed genotyping of bisulfite-converted genomic DNA. Upon treatment with bisulfite, unmethylated cytosine bases are converted to uracil, while methylated cytosine bases remain unchanged. These chemically-differentiated loci are interrogated using two site-specific probes (DNA oligonucleotides), one designed for the methylated locus and one designed for the unmethylated locus of a particular genomic region. The probes incorporate labelled ddNTP, which is subsequently stained with a fluorescent reagent. Level of methylation for the interrogated locus can be determined by calculating the ratio of the fluorescent signals from the methylated vs unmethylated sites. Other methodologies for the establishment of DNA methylation signatures are known for the expert and include Methylation-Specific PCR (MSP), ChIP-on-chip assays, Pyrosequencing of bisulfite treated DNA, and High Resolution Melt Analysis (HRM or HRMA).

The method for HCC prognosis of the invention has been validated in a cohort of samples after determining its essentialities in a training set cohort. Additionally, the data obtained from the method correlated with the data retrievable from other prognostic methods for this cancer, including data from differential mRNA transcription between subjects with good and bad HCC prognosis, as well as correlating with other clinical variables reported as outcome predictors in HCC (gender, age, etiology of liver disease, tumor size, serum albumin and bilirubin, platelet count, serum alpha-fetoprotein (AFP), cancer stage (in particular BCLC stage), microvascular invasion, satellites and degree of differentiation of the tumor).

Also as will be illustrated below and of particular interest is the fact that the DNA methylation signature obtained with these methylation analysis of the above identified cytosines (from CpG sites) allows calculating a value called DNA methylation based mortality index (MI), which is a value that allows correct split of patients/subjects between those of good and bad prognosis for the disease. This MI further implies the advantage of informing the clinician in a precise way of the gravity of a particular case. Fast determination of the prognosis aids in the taking of decisions relating to the most appropriate medical regimen for a particular subject.

For this reason, in a second aspect the invention relates also to methods of deciding and/or recommending whether to initiate a medical regimen of a subject suffering HCC, which methods comprise the steps of providing from an isolated sample of a subject a DNA methylation signature and determining the prognosis of hepatocellular carcinoma by means of a method as defined above; and wherein if the subject is determined as having a bad prognosis, then a specific medical regimen comprising intensive monitoring after resection may be recommended. For intensive monitoring is to be understood that follow-on of the patient is performed at short interval periods.

This second aspect is the direct consequence of the method of prognosis of the invention according to the first aspect, since after having a correct classification of the subject, the clinician may decide or may recommend a particular therapeutically regimen, avoiding extra costs of non-useful therapeutics or regimens and saving time, a valorous aspect for the treated HCC subjects.

The invention also encompasses, as a third aspect, the use of means for the prognosis of hepatocellular carcinoma according to the method disclosed in the first aspect, said means comprising DNA oligonucleotides suitable for determining DNA methylation signatures of a set of cytosines of CpG sites.

These means allow providing the informative DNA methylation signature from which a prognosis is established.

As will be depicted in more detail below, said DNA oligonucleotides are indeed probes or primers comprising sequences being complementary either to methylated locus or to unmethylated locus of the CpG site cytosines. Thus, these oligonucleotides are suitable to detect the CpG sites disclosed in the present invention. As above exposed any of said CpG sites can be identified by means of the Illumina®'s CpG Loci Identification which consist in any of the sequences SEQ ID NO: 1 to 36.

Forms also part of the invention the use of the DNA methylation signature, defined by the qualitative and quantitative methylation analysis of the set of cytosines of CpG sites comprising the cytosines identified above, as biomarker for the prognosis of hepatocellular carcinoma in an isolated sample.

## BRIEF DESCRIPTION OF THE DRAWINGS

FIG. 1 shows different boxplots (with over-imposed individual values) for different clinical and pathological variables known to be prognostic in HCC and the DNA-methylation-based mortality index (MI). FIG. 1A shows vascular invasion (absent or present), FIG. 1B shows multinodularity (single or multinodular), FIG. 1C shows satellite tumors (absent or present), FIG. 1D shows the Barcelona Clinic Liver Cancer (BCLC) staging (ranging from 0 to stages A, B or C), FIG. 1E shows alpha-fetoprotein levels (AFP in mg/dl),

FIG. 1F shows bilirubin levels (mg/dl in abscissa), FIG. 1G shows the platelet count (in number/$\mu$l), and FIG. 1H the albumin levels ( g/dl indicated in abscissa).

FIG. 2A is a plot with the predicted hazard ratio (HR from 0 to 100 %, Y-axis) for survival based on the DNA methylation based mortality index (MI) (X-axis) in a training set. FIG. 2B and 2C are Kaplan-Meier plots for outcome analysis in the training set, showing respectively the overall survival (OS) and the recurrence (R) (Y-axis) along time (T in months (m), X-axis).

FIG. 3 is also a Kaplan-Meier plot for the survival (S) along time (T in months (m)) in a validation cohort.

## DETAILED DESCRIPTION OF THE INVENTION

All terms as used herein, unless otherwise stated, shall be understood in their ordinary meaning as known in the art. Other more specific definitions for certain terms as used in the present application are as set forth below and are intended to apply uniformly throughout the description and claims unless an otherwise expressly set out definition provides a broader definition.

In the sense of the present invention, the expression "DNA methylation signature", "methylation-based signature" or "DNA methylation pattern" (used herewith as synonymous expressions) relates to the qualitative and quantitative methylation in a particular nucleotide, nucleotide sequence or sequence set (group of sequences). DNA methylation is a biochemical process where a methyl group is added to the cytosine or adenine DNA nucleotides. Most particularly, methylation is usually found in CpG islands, which are regions with a high frequency of CpG sites. CpG sites or CG sites are also regions of DNA (or DNA genomic sequences) where a cytosine nucleotide occurs next to a guanine nucleotide in the linear sequence of bases along its length. Cytosines in CpG dinucleotides can be methylated to form 5-methylcytosine. The CpG sites are well-defined genomic regions and they are indexed in database giving to said regions an identification number (ID) as cg_number, according to Illumina®'s CpG Loci Identification wherein flanking sequences regions around the CpG dinucleotide are used to generate unique CpG cluster IDs (cg_number).

When methylation of a sequence is analysed, it is determined by any means where methylation occurs (position in the sequence) and at what extent it occurs (that is, the percentage of methylated cytosine of a particular sequence in an analysed sample), which is the methylation level. It is widely accepted in the quantification of methylation or in determining the "methylation level" to give a 0 value score from completely unmethylated cytosines and a value of 1 to fully or completely methylated cytosines of a CpG site in a genomic sequence. This score is commonly named the β-value score, and it is calculated according to formula (I):

$$\beta\text{-value}_{Cyt} = \max(y_{methCyt}, 0) / [\max(y_{unmethCyt}, 0) + \max(y_{methCyt}, 0)] \quad (I),$$

wherein max $y_{methCyt}$ is the maximal signal intensity detected for a methylated cytosine in the CpG site set; and max $y_{unmethCyt}$ is the maximal signal detected for an unmethylated cytosine in the CpG site set.

Then, from a set of sequences including cytosines of CpG sites, a particular "image" of the methylation state can be retrieved showing methylated sequences (qualitative) and the degree (quantitative) of methylation of these sequences in a particular sample.

The parameter herewith called "DNA methylation based mortality index (MI)" is a value calculated from the DNA methylation signature by dropping down the signature from an isolated subject of the sample subject to a random survival forest (RSF) model for outcome prediction (prognosis). From the RSF a parameter named ensemble cumulative hazard function is determined and a mortality index is obtained as a weighted sum over the ensemble cumulative hazard function (CHF), weighted by the number of events at the different time points along analysis time of a training cohort for preparing the model.

"Overall survival" expresses the time between surgical resection and death of any cause or last follow-up, whereas "Cancer-related survival" is the time between resection to HCC-related death. An estimated value of these overall and cancer-related survivals means the most likely time from surgical resection to death.

"Time to recurrence" is the time between resection and the radiological evidence of first tumor recurrence.

The expression "reference DNA methylation signature" or "reference signature" (used herewith interchangeably) is to be understood as the level of methylation obtained from a group of samples previously analysed and in which, at least each of the cytosines have been analysed in terms of detecting methylation and the quantification of said methylation. The samples are taken from a subject or group of subjects wherein the presence, absence, stage or course of the disease has been determined previously. Likewise, the "Reference value" referred to in the methods of the invention, can be a reference DNA methylation based mortality index (MI) from which a particular correlation with the disease (i.e. HCC) has been previously performed, also with a subject or group of subjects wherein the presence, absence, stage or course of the disease has been determined previously. In the same way, the concept "reference" may encompass a decision rule to which a methylation analysis (qualitative and quantitative) is contextualized to get an associated particular outcome (overall survival, time to recurrence, etc.). As a "reference" also an image may be used, said image derivable from the analysis of all the cytosines listed above by means of arrays including oligonucleotides for such purpose. The skilled person in the art, making use of the general knowledge, is able to choose the subject or group of subjects more adequate for obtaining the control references for each of the methods of the invention. Generally, the reference signature, the reference value and/or any decision rule are references allowing splitting tested subjects into two or more groups, according to the prognostic (most likely outcome) of the disease.

Methods for obtaining the reference value/signature from the group of subjects are well-known in the state of the art. In the present case, and in case a DNA methylation based mortality index is computed, inventors propose the use of the Random Survival Forest (RSF) model. This model estimates a cumulative hazard function (CHF) from survival trees (Ishwaran et al., "Consistency of Random Survival Forests", Statistics and Probability Letters - 2010, Vol. No.: 80, pp.: 1056-1064).

As used herein, the expression "good prognosis" means that the patients can expect a likely survival time higher than that of patients with bad prognosis. In general terms "bad prognosis" would estimate that the mean of patient's survival time is lower than 24 months, while patients with good prognosis are those predicted of having a mean of survival time higher than 60 months.

When in the present invention there are used the expressions "genomic sequences" or "genomic regions", sometimes referred as "probes" when they are in a DNA array, relate to fragments of the genome that are to be analysed. More in particular they relate to CpG sites including the cytosines which methylation is to be determined.

In a particular embodiment of the first aspect of the invention, the method comprises comparing the DNA methylation signature of the subject with a DNA methylation signature (also named reference control signature), and a bad prognosis is determined if the DNA methylation signature is within a range of DNA methylation signature indicative of bad prognosis.

The reference DNA methylation signature is in a particular embodiment a DNA methylation signature of a cohort of subjects or of a subject suffering from HCC. Alternatively, the reference DNA methylation signature is given as the result of an RSF, that is, as the range of mortality index (MI) values derived from the processing of signatures of a cohort of subjects, in particular a training cohort of subjects suffering from HCC.

In yet another particular embodiment, the set of cytosines of CpG sites consists in:

Cytosine at position 25062754 of human Chromosome 20;
Cytosines at positions 67571354, 30022699, 2932904 and 67313014 of human chromosome 16;
Cytosine at position 112194776 of human chromosome 6;
Cytosines at position 147127012, 159943073 and 140660432 of human chromosome 3;
Cytosines at position 47797415, 26624823, 45236267 and 74648928 of human chromosome 2;
Cytosine at position 38747378 of human chromosome 19;
Cytosines at position 94928855 and 8559999 of human chromosome 8;
Cytosine at position 34443010 of human chromosome 21;
Cytosines at position 24899054, 23821445, and 69726620 of human chromosome 14;
Cytosine at position 107188416 of human chromosome 13;
Cytosine at position 48908469 of human chromosome 4;
Cytosines at position 108096745, 95401692, 155249746 and 5633086 of human chromosome 7;
Cytosines at position 75671378 and 97803058 of human chromosome 10; Cytosine at position 83378614 of human chromosome 15;
Cytosines at position 24130633 and 32847789 of human chromosome 18; Cytosine at position 114632040 of human chromosome 5;
Cytosines at positions 66193820, 21279621 and 79197398 of human chromosome 17; and
Cytosine at position 35639656 of human chromosome 11.

These cytosines are in known identified CpG sites, herewith also defined by sequences SEQ IDs NO: 1 to 36 or by the Illumina®'s CpG Loci Identification cg_number notation, as depicted in Table A below. Most of these sequences are located in genome regions including promoter gene regions.

Table A below shows the 36 genomic regions (CpG sequences including cytosines of interest) disclosed in the method of the invention, with a bold and underlined cytosine corresponding to the putative methylated cytosine in this site:

Table A

| Cytosine position in the chromosome (1) | Hum Chr | CpG site identification Nr (Illumina) / Gene symbol | SEQ ID NO: | DNA sequence |
|---|---|---|---|---|
| 25062754 | 20 | cg00043788 / VSX1 | 1 | GCTAGTGCGCCCGTCGGAAAGCGAGTCCCGGCCGGTCATGGTTCCTTAGCAAGCAAGGCG[**<u>C</u>**G]AGCCTCTCTGGATCCCGTTTGCGGAGGGCCCAGCTTAGAGGAAGCTTTATAGGATTGGGC |
| 67571354 | 16 | cg00586537 / FAM65A | 2 | CGGTCCCGCCCCATCCAGGCGGGCTGAGTCAGGCGGCAGGAACTGGGCGGGGGGCGGCGC[**<u>C</u>**G]GGAGGAGCCGAAGCCGAGCCAGAGCCGCTGGGAGCGAGCCCGGAGCCCAGCCGGGCGGCT |
| 112194776 | 6 | cg00696540 / FYN | 3 | CCGGCTGCCCTATCAAAGAGCCCCTTGGGAGCTGCGAGCCGCAACCATTGGCGCCTGGGG[**<u>C</u>**G]TGGGCGGGACGGCAGTAGGTGGGGCCGCCGAGTGGGTTGAGCGTTACTGTGGCAGCGGGC |
| 147127012 | 3 | cg01227537 / ZIC1 | 4 | GATGTCAAGCGCTTTACAATACCTGGGATTGATGAGGCGGGCGGGCCAATGAGCTGCGCG[**<u>C</u>**G]GCGCCTCGGCGCGCCCTCCGTTGGCGCGGCGGCTGAGGGCGGGGGGAATGCGGGCGCACC |
| 47797415 | 2 | cg02318629 / KCNK12 | 5 | AAGCGGCCGGTGTCCTCGTTGAGGTGCGAACGGCGGCAGCAGCAGCAGCAGCAGGAGGGG[**<u>C</u>**G]CGGCAGGCGGCGGCGGCTACGGCGGGGCGGGGGCCGGGGGCTGCGGGAGGACATGGTCCG |

(continued)

| Cytosine position in the chromosome (1) | Hum Chr | CpG site identification Nr (Illumina) / Gene symbol | SEQ ID NO: | DNA sequence |
|---|---|---|---|---|
| 38747378 | 19 | cg02571816 / PPP1R14A | 6 | GCGGGGTGGGGGGCTGTTGGTGCGGGAGCGCCTGCGCCGCTGTCGCTGTAGAAGGTCGAG[**C**G]GTCACTTGTTCCTTCGTTCATTCATTCACTCATCCATCCATTTGTTTGTTCACTCATTCC |
| 94928855 | 8 | cg03578886 / PDP1 | 7 | ACATTGGAGCCGGCCCAGGCGCCTCCCCTCTCCCGACCCCGCAGCCTGCTCAGCCAACAC[**C**G]CCGAGCTCCACAGACCACCGCTCCCTAGCGCCGCGCAGCCACACCTTCCACTCGCTGGGG |
| 34443010 | 21 | cg03732762 / OLIG1 | 8 | TGCCCTACTCAGCGGCGCACTGCCAGGGCGCGCCCGGCCGCAAGCTCTCCAAGATAGCCA[**C**G]CTGCTGCTCGCCCGCAACTACATCCTACTGCTGGGCAGCTCGCTGCAGGAGCTGCGCCGC |
| 30022699 | 16 | cg03920233 / DOC2A | 9 | TGGCACACTTACCCGGAGAACTTCGCACACACACGCACTCGCAAACACGCGGGCTCCCTG[**C**G]CCACCAGGAAGCAGCCGCCAGAATCGCCGGGTGTTCCCCAACTACTCCTGGGAGACCTGG |
| 24899054 | 14 | cg04274978 / CBLN3; KHNYN | 10 | GGAGGGCTGGTGAGAAGCGCAAGGGGCGAGCCCTGGAGCCGCCGAGGGGACTAGGCCGAG[CG]GGCCCGTCGGGGCCTCTGGGCGCGGCGGCGGGGTTGGGAGGAGGGCGCGCAGCCGGGAGG |
| 107188416 | 13 | cg04321866 / EFNB2 | 11 | CCCCTCGGCGGAATCAGAGGGCGCGGCTTTCCCTCGAGCAGTGCGGAAGGCAGCAGGCTC[**C**G]CTCGGCGTCCCTTCTCCGCAGATGCGGCGCTGCCCCGCGTGCAAGACTTGCACTCCGCGC |
| 48908469 | 4 | cg04961553 / OCIAD2 | 12 | ACGGGGGACACTGCACTTTCTGATGCGGTCGGGGAAGCCGGGAGTCAGCCTACTGGAGTC[**C**G]CTGCCATCCCTGGCCAGAAGCAGCCAGCGCCACCCCAAGCTCCCCAGCCCCTCGCCCGTG |
| 108096745 | 7 | cg05412664 / NRCAM | 13 | ACGTTCTCGCGCCGCGCCCTCCGCTCAGCTCTGGCGCGACTGCCCAGGACCCTGGACCGC[**C**G]GTGTCCTCCGTTCTCGACGAAGCTATCCCCTCTGGCTGCTCCAGCCAGGCGGCCCCTGCG |
| 75671378 | 10 | cg06521280 / C10orf55; PLAU | 14 | GAGCCCTGCTGGCGCGCCTGCTTCTCTGCGTCCTGGTCGTGAGCGACTCCAAAGTGAGTG[**C**G]CTCTTGCTTTGACTGATGCTGCCCAAGGACCTCTGATCAGCACCAGGGGAGAGGAGGGGC |
| 8559999 | 8 | cg06671706 / CLDN23 | 15 | ATGGTGTTGGCGCCCTGCGGGCTCCTGCTCAACCTGACCGGCACCCTGGCGCCCGGCTGG[**C**G]GCTGGTGAAGGGCTTCCTGAACCAGCCAGTGGACGTGGAGTTGTACCAGGGCCTGTGGGA |
| 95401692 | 7 | cg07732116 / DYNC1/1 | 16 | GCAGGAGCTCCCAGAATATTGAAGCTCTTCTCTTTGCAGCAACTGAGGTCTAAGTCCTAC[**C**G]ACGTTAGAGGGATAGACGAATTGACCTTTCCAGGTCCTCTGCTCGCCACCAGCCTACGGA |

(continued)

| Cytosine position in the chromosome (1) | Hum Chr | CpG site identification Nr (Illumina) / Gene symbol | SEQ ID NO: | DNA sequence |
|---|---|---|---|---|
| 97803058 | 10 | cg08379212 / CCNJ | 17 | TGGCCTAACTGGATTGGTCACTGCAGTGATCCACCTTACAATTTCTCCGGCGCTCTCTGC[CG]ACCTCAGCCGGCGATCAGGCCGGCTTCGCAGTAGGTGGCGGCTGAGCGCCCCGGCAACCG |
| 23821445 | 14 | cg09321747 / SLC22A17 | 18 | CAGCGGGGGCGCCAGCGTGAAGATGGGGTCCGAGGCCATGCCCAGAGCCACGAAGAGCAC[CG]GCAGGCAGCAGAGGCCGAGCTGCAGCTGCTGGCCGCCGCCCAGCGCCCCCACCTGGGCGA |
| 83378614 | 15 | cg09472203 / AP3B2 | 19 | GCGAAGGCGGCGGCGCGGCAGGGGTTCAGCAGAGGCTGCAGTGTGCAGCGGCGGAGGCTG[CG]CGCGGATTTCTCAATCAGGGCCGCGCGCTGAGGTCTTAAAGGCACCGGCGCCAGCCGAGC |
| 155249746 | 7 | cg10481660 / EN2 | 20 | ATAAGTGTCTGCAGGAGGAGTGTCCTGCGCGCCAGCTCTGCGTTTAAGACAGGAAGCTGC[CG]GGTTACCGAGTCAAATGGGAGTGACACTATTCCTCTCCATCAGCAAGGAAAGCGGACCAC |
| 24130633 | 18 | cg12881557 / KCTD1 | 21 | GCTCTCATCCGCCCGGCTCTCCGCGGAGAACCGTCCGGGAATCGTCCCCGAGGTGCGGCG[CG]GGAGGTGGCCGGCTTTGTTCACAATGCACTGCCCGCCGCACCCGCCCTGGACACGTGCAG |
| 26624823 | 2 | cg13414212 / C2orf39 | 22 | TGACCGCTCTCCCTGGCAACGGTTTGTTCCTAGCAACCAGCCTGAGGTCTGGAGGTGGTG[CG]GAGGGAGCCGCCTAGGGACCAGGGACTCCTGCCATGAATCCGCCGGGGTCCCTAGAGGCC |
| 45236267 | 2 | cg15019790 / SIX2 | 23 | CCACTTGCTCCTGCGTGAAGCCGAAGGTGGGCAGCATGGACATGGTGCCGGCTGCGTCCC[CG]CCCGCCCGCGCGCGCCCTCACCGGGCCGCGCGGTCCCGCATGGGAGCTTCCTCGCCGGGC |
| 69726620 | 14 | cg15065139 / GALNTL1 | 24 | TGCCGGGCGTTCGGTCACCTAGCGGTGGGAGCCGAGGCTGGCTTTGGCCAGTCGCCATTC[CG]CCGCGGGCGTCTGTCTCGCCGGGTTCCCGGGCGCGGGAGGAAGGCCCGCGCCGCTTCCCA |
| 114632040 | 5 | cg16113530 / CCDC112 | 25 | AGAGCAACGCCCAGCGCGTGCCAGGCCCCGAGCAGGGGAGGCGAACGCCGCGCCTCCCGC[CG]GCGCTGCAGAGGGCACCTGTTCAGCCAGGGCCTGCAGCCCCTCGCCTGCCGCCAGAGCAG |
| 74648928 | 2 | cg17157630 / WDR54 | 26 | CGTCGTCTCTATGGTGGCGGCGGATTTGGAGGGACCCTACGAACCAGGAGTCAGGCGAGC[CG]ATCTGGGGCTGCAGGTGTTACCTCTGATCTAGGCCGGGGGCTTCAGGGATCCGAGCCGAG |
| 66193820 | 17 | cg17179589 / LOC440461 | 27 | GGGGAAGGGGCGCCGGGGCAGTGTCCTCCTTGGGGTTTGTTGGTCCAGCTCTGGGCAGCT[CG]ATGACCCCGCCTCCGGGACCCAGTGTGCTTAGTGACTCAGTTTACACTTTTTCCTCGTCG |

(continued)

| Cytosine position in the chromosome (1) | Hum Chr | CpG site identification Nr (Illumina) / Gene symbol | SEQ ID NO: | DNA sequence |
|---|---|---|---|---|
| 159943073 | 3 | cg18703913 / LOC401097 | 28 | AGAGCGTTAACCCTTCCAGTCCCAGAGAGCGACAAGGCGGGGGAGGAAAAACGCGCCGGC[**C**G]GGGCCAAGATGCCCATGGCAGCCCCGCGCGGGCTGCCTCTGACATTTAGGGAGGCTCCGG |
| 2932904 | 16 | cg21085535 / FLYWCH2 | 29 | GTCAAGGATGCAGAGCTTAAAGCAAGGCAGTGGCTTCCATGAGCCTAAACACCTTCCCTC[**C**G]CACGCTCGGTATCGCGACGGGGGAGAGCAAAACCTTTAAACAAATGCTCCAGTTTTCCCC |
| 5633086 | 7 | cg22447539 / FSCN1 | 30 | GCACCTGAGCGCGCGGCCGGCCGACGAGATCGCCGTGGACCGCGACGTGCCCTGGGGCGT[**C**G]ACTCGCTCATCACCCTCGCCTTCCAGGACCAGCGCTACAGCGTGCAGACCGCCGACCACC |
| 21279621 | 17 | cg22639895 / KCNJ12 | 31 | CGGGACTAGGAGGGGCTCGGGGCCAGGCCGGAGCCGTGGTAGGCTGGTGCAGCGGCTGCG[**C**G]GCGCGGCGCGGCGCGGGGCGGGCGTGCTCTCCGCGGAGAGTTAGAGGAGTTGCCGAGCTG |
| 35639656 | 11 | cg23083315 / FJX1 | 32 | GAGGTGGAAATCTGTCTGAAGTTTGCAGGAGACCCGCGCTGCCTAGGCAAAGGGACCCGG[**C**G]ACCAAGACACCCCCGCGAGGGCCCAGGGCGCGTCCCTCCCGCCAGGCCCCAGACTCCGGC |
| 32847789 | 18 | cg23624808 / ZNF3970S | 33 | ATTCAAACATATTTTTCCTCGTTATGTCGCGGGCTGGTATACCATCGAGAAAATAAAAAC[**C**G]AGAACCCATGATCTCTACATTTTGAGAGGAAGAGGAGCTGACCACGCTCACCGCGGACGT |
| 140660432 | 3 | cg23664775 / SLC25A36 | 34 | CCGCCAGTTCCCTGTTCAGGAGAGTGCAGCCGTGATTGGCGCGTTCACTCAGGCCAGGGG[**C**G]CCCCAGGTCTCAGGTGCTAGGAGCGCGGTGACCTACCACCCTCCGGGCACGCGTTCTGGC |
| 79197398 | 17 | cg26527638 / AZI1 | 35 | TCCCTGACCTCCTTCCCTTCGCGCTGCTTTGTTGTTTTTTTTCCTTCTCCAAAGTTTTAA[**C**G]CTGCGCCCCGTGCGTAGCCGCTCTGTGACTCCCCCTCTCTCCGGGAGCCCTGGGGATTCC |
| 67313014 | 16 | cg26793648 / PLEKHG4 | 36 | TACACAGAAGGCCTTCCCCTCCCGTCGGCTCATTCAGCCGCGGAAGCCCCTGGGAGACCC[**C**G]CCCCGCCCCGTCCCGCGGTGCCCCCCGCGCACTCACCCTCGCTCACCCACGCGGCCTTCC |

Hum Chr: Human chromosome
(1): positions in human chromosomes according to the chromosome map and sequence entries of database UCSC Genome Browser on Human February 2009, GRCh37/hg19 assembly of the University of California Santa Cruz (UCSC).

In a particular embodiment of the first aspect of the invention, optionally in combination with any of the embodiments above or below, the qualitative and quantitative methylation of the set of cytosines of CpG sites defining a DNA methylation signature from the sample, is compared with a reference methylation signature, and a bad prognosis is determined if the DNA methylation signature of the sample is within a range of reference signature indicative of bad prognosis.

This reference DNA signature may in particular be a reference value in case a parameter is computed (such as a mortality index) from the individual methylation presence or absence and from the methylation levels of the in CpG site cytosines analysed. It can also be a reference image, in the particular case the analysis of all the cytosines listed above is performed by means of arrays including oligonucleotides, said oligonucleotides giving a particular signal that is translated into an image. Examples of images for the analysis of methylation include the coloured images from oligonucleotide arrays, wherein colour is the result of a fluorescent or chemiluminiscent compound. Creation of images of the arrays can be performed with appropriate scanners (such as Illumina iScan SQ). Intensity of the signal can be computed with particular software packages (such as the Illumina GenomeStudio methylation module).

In a more preferred embodiment of the method, the qualitative and quantitative methylation analysis comprises determining if in the isolated sample each cytosine of the set is methylated or not, and if methylated determining the level of methylation in said position, expressed as a β-value ranging from 0 to 1, by means of formula (I):

$$\beta\text{-value}_{Cyt} = \max(y_{methCyt}, 0) / [\max(y_{unmethCyt}, 0) + \max(y_{methCyt}, 0)] \quad (I),$$

wherein max $y_{methCyt}$ is the maximal signal intensity detected for a methylated cytosine in the CpG site set; and max $y_{unmethCyt}$ is the maximal signal detected for an unmethylated cytosine in the CpG site set.

In yet a more particular embodiment, optionally in combination with any embodiment above or below, the method comprises obtaining a DNA methylation based mortality index (MI) by means of the following steps:

(a) dropping down the DNA methylation signature from the isolated sample of a subject to a random survival forest (RSF) model for prognosis (i.e. outcome prediction), wherein said RSF model is built using Ntrees from the quantitative methylation analysis of a set of cytosines as defined above, said set of cytosines analysed in isolated samples of a training cohort consisting of subjects suffering from HCC;
(b) deriving a cumulative hazard function (CHF) from each of the trees of the model;
(c) determining an ensemble cumulative hazard function as an average of CHF of each tree; and
(d) obtaining a DNA methylation based mortality index as a weighted sum over the ensemble cumulative hazard function (CHF), weighted by the number of events at the different time points along analysis time of the training cohort.

In a preferred embodiment the Ntrees for building the model are 1000 trees. In another preferred embodiment, the numbers of events correspond to the number of deaths in a group at a particular time point, generally at a month of the time period of the analysis of the training cohort, used for building the RSF model.

The CHF corresponds to the accumulation of the "hazard" over time, and it is generally estimated by the formula (II) in a RSF model and for a node h and time t:

$$\hat{H}_h(t) = \sum_{t_{l,h} \leq t} \frac{d_{l,h}}{Y_{l,h}}. \quad (II)$$

wherein $H_h(t)$ is the estimated hazard function for a terminal node h of the tree, said terminal node defined by a node in which the tree reaches a saturation point, which means that no new daughters can be formed because of the criterion that each node must contain a minimum of $d_0>0$ unique deaths (events); t is the time; $d_{l,h}$ is the number of deaths in the node; and $Y_{i,h}$ are the individuals at risk at time $t_{l,h}$.

The DNA methylation based mortality index (MI) is thus defined from the cumulative hazard function (CHF) as the expected value for the CHF summed over the ensemble, and weighted by the number of events at the different time points. In plain words, the MI for a sample *s* represents the estimation of the average number of individuals in the data set that would be dead provided they had the same methylation level as *s*. Thus, higher MI implies higher risk.

A complete description of the MI measure, and the random survival forests (RSF) method, is well described in Ishwaran H, Kogalur U, Blackstone E, Lauer M: Random survival forests. Ann Appl Stat-2008; 2: 841-860.

Thus, from the methylation level of each of the cytosines listed above a β-value$_{Cyt}$ is obtained, which as a whole determines a DNA methylation signature. This signature is contrasted or put in context (dropped down) to the RSF model from the training cohort until it matches with a DNA methylation based mortality index.

With the MI derived from the RSF model built with the training cohort, the training set can be split in *n* risk groups based on percentiles and matching with a predetermined prognosis. A test isolated sample for which an MI is also computed dropping down the signature to said RSF, will fit or accommodate to one of those risk groups with an associated prognosis.

In a particular embodiment, if the DNA methylation based mortality index obtained for a test sample is over or above a cut-off value, said cut-off determined by splitting the samples of the subjects with the lowest error, then the prognosis of the test sample can be done on the basis of said DNA methylation based mortality index with the lowest error and with high sensitivity and specificity. Alternatively, if the DNA methylation based mortality index (MI) obtained for a test sample is within a range of a reference MI indicative of a particular condition (for example bad prognosis defined as low overall survival, recurrence, etc.), the sample is attributed said condition.

Inventors surprisingly found that a DNA methylation signature derived from the qualitative and quantitative analysis of at least the 36 cytosines above indicated, allows good classification of samples with the lowest error, independently of the cause of HCC (hepatitis B or C, alcoholism, etc.). Therefore, the invention proposes for the first time a simplified validated methylation pattern with meaningful prognostic value for any sample HCC etiology.

A DNA methylation signature with a combination of two or more of the 36 cytosines, in particular at least twelve and more particularly a combination of at least a number of cytosines selected from eighteen, twenty, twenty-two, twenty-four, twenty-six, twenty-eight, thirty, thirty-two, and thirty-four serves for predicting with a low error the outcome (prognosis) of a test sample.

In yet another particular embodiment of the method of the invention, the subject is a surgically resected subject, which means that liver tumor has been removed. In this particular type of subject prognosis establishment helps to identify in an easy mode if recurrence of cancer occurred with a high probability.

The method of the invention provides information of the prognosis of the subject in terms of indicating or predicting the most probable clinical evolution or outcome of the disease. In other words, the prognosis is materialized in a clinical parameter and/or condition that correlate with the most probable outcome or evolution of the patient subject. Thus, in another particular embodiment, the prognosis includes indicating or predicting the probability of recurrence of HCC, and/or the estimated overall survival, and/or the subtype of cancer, and/or the cancer-related survival from the isolated sample of the subject.

In a more particular embodiment, the prognosis includes indicating probability of recurrence of HCC, which means the probability of further suffering from HCC or of any satellite tumor thereof.

Also in another more particular embodiment, the prognosis includes indicating the estimated overall survival.

Yet in also another more particular embodiment, the prognosis includes indicating or diagnosing the subtype of bad-prognosis stage cancer the subject is suffering (i.e. bad-prognosis stage HCC). This is an additional particularly interesting hit of the method of the invention, since a cancer subtype stemmed from progenitor cell origin may be simultaneously diagnosed or suspected in the tested HCC samples of the patients only with the analysis of the 36 cytosines indicated. In particular, an HCC subtype that is enriched in mRNA signatures capturing progenitor cell features. Early detection of this phenotype allows fast focusing on this cancer type of bad prognosis.

In another particular embodiment of the method, the analysis of cytosines of CpG sites is performed with a set of DNA oligonucleotides that are suitable to detect the sequences comprising SEQ ID NO: 1 to 36, which means that they are partially or totally complementary to any of sequences consisting in said SEQ ID NO: 1 to 36. These sequences 1 to 36 correspond, as indicated in Table A, to genomic fragments known and duly identified as CpG sites. The oligonucleotides (with a length from 15 to 30 nucleotides) are adapted to detect either the methylated locus or the unmethylated locus of said CpG sites cytosines. Complementary sequences are paired by hydridization at a temperature comprised generally from 40 °C to 50 °C for a time comprised from 8 to 20 hours.

For the expression "adapted to detect either the methylated or un-methylated locus" is to be understood that the DNA oligonucleotides are capable of detecting, for example by hybridization, either the methylated cytosine or the unmethylated cytosine of a CpG site defined by a genomic sequence comprising any of SEQ ID NOs: 1 to 36. Detection is made since DNA oligonucleotides act as primers and/or probes and allow amplification of any of the above genomic sequences (CpG sites including the cytosine of interest). Particularly, the DNA oligonucleotides may act as probes for detecting methylation position in any of SEQ ID NOs: 1 to 36. The quantitative analysis of methylation in a site may be performed by calculating the ratio of a signal (such as fluorescence) provided from the DNA oligonucleotides adapted to detect the un-methylated locus, and the signal provided from the DNA oligonucleotides adapted to detect methylated locus in that particular CpG site cytosine. An example of this is the β-value disclosed above.

The invention relates also to the use of means comprising said DNA oligonucleotides suitable for the analysis of the qualitative and quantitative methylation of at least the cytosines of CpG sites comprising or consisting in any of SEQ ID NOs: 1 to 36, for the prognosis of hepatocellular carcinoma in any of the methods disclosed above.

In a particular embodiment, the means comprising DNA oligonucleotides form part of a DNA array.

In a particular embodiment the use of these means relates thus to the use of DNA arrays or to kits for DNA analysis including as reagents the DNA oligonucleotides that are able to detect methylation in the above mentioned CpG sites (namely in the cytosines of these CpG sites).

An example of commercial arrays including DNA oligonucleotides suitable to analyse methylation levels of at least the CpG sites comprising or consisting in any of SEQ ID NOs: 1 to 36 is the Illumina® Infinium HumanMethylation450 BeadChip array.

Oligonucleotides in the present invention are to be understood according to the widely accepted meaning, as the short (with a length from 10 to 60 nucleotides, or as above exposed preferably from 15 to 30 nucleotides), single-stranded DNA or RNA molecules that have a wide range of applications in genetic testing. They are commonly made in the laboratory by solid-phase chemical synthesis. They are characterized by the sequence of nucleotide residues that make up the entire molecule. Oligonucleotides readily bind, in a sequence-specific manner, to their respective complementary oligonucleotides, DNA, or RNA to form duplexes.

In another particular embodiment, optionally in combination with any aspect or embodiment of the invention disclosed above or below, the isolated sample of the subject for determining the DNA methylation signature is a sample selected from the group consisting of a liver biopsy, blood (including whole blood, serum or plasma), urine and saliva. In a preferred embodiment, the isolated sample is a liver biopsy. Most preferably, it is a tumor liver biopsy. Indeed, any isolated sample of the subject from which DNA can be extracted for further methylation analysis will be useful. Most preferably, it is a sample from which tumor cells may be isolated and analysed.

Yet in another preferred embodiment, the liver biopsy is a tumor liver biopsy and an independent non-tumor tissue (from liver biopsy) of the same subject. By means of this sampling, DNA methylation signature of both tissues (tumor and non-tumor) can be compared avoiding any inter-individual variability.

In another particular embodiment, optionally in combination with any aspect or embodiment above or below, the method for the prognosis of HCC further comprises determining in the isolated sample of the subject an RNA-transcription signature resulting from the qualitative and quantitative analysis of transcription of an RNA selected from the group consisting of miRNAs, mRNA and mixtures thereof.

Examples of commercially available kits and arrays for the RNA signature include the Affymetrix Human Genome U219 Array Plate.

In a particular embodiment, said RNA transcription signature results from the analysis of the levels or amounts of at least one of the non-coding micro RNA selected from miR-3193, miR-27b-star, miR-422a, miR-378c, miR-938 and combinations thereof, and in particular a combination of all. In a most particular embodiment, the RNA transcription signature comprises further the analysis of the small nucleolar RNA known as SNORD126 (also identified as miR-1201).

Any of the herewith disclosed in vitro methods, having in common that all give data about the prognosis (or outcome) of HCC, may in any particular embodiment or combination of embodiments include a further step of collecting and/or providing and/or saving data derived from previous steps in a data carrier. Thus, the invention also encompasses any data carrier with the prognosis data directly obtained from any of the methods of the invention.

In the sense of the invention a "data carrier" is to be understood as any means that contain meaningful information data for the prognosis of HCC. Examples of data carrier are printed copies of paper correlating the prognosis of the disease with the DNA-methylation signature derived from the analysis, in the isolated sample of a patient, of at least the CpG site cytosines disclosed above and comprised in the sequences SEQ IDs NO: 1 to 36. The carrier may also be any entity or device capable of carrying the prognosis data. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the prognosis data are embodied in a signal that may be conveyed directly by a cable or other device or means, the carrier may be constituted by such cable or other device or means. Other carriers relate to USB devices and computer archives.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

EXAMPLES

Next examples aim to illustrate the applicability of the methods for the prognosis of HCC of the invention.

The method for the prognosis of the invention, comprising determining the DNA methylation signature from at least the set/group of sequences consisting in SEQ ID NOs: 1 to 36, which includes the 36 cytosines of interest, was developed with a training set of patients and validated with another independent set.

Human samples:

The study included samples from 331 surgically resected HCC and 19 non-tumoral tissues including 9 cirrhosis and 10 normal livers. The training set (n=248) were liver tumor biopsy samples obtained from the HCC Genomic Consortium: IRCCS Istituto Nazionale Tumori, Milan (n=217) and Hosptal Clinic, Barcelona (n=31). Methylome profiling (including level of methylation and location of methylation) was performed in the 248 samples with the Illumina® Infinium HumanMethylation450 BeadChip array that interrogates 485000 CpG sites and covers 96 % of the known CpG island. Finally 221 HCC samples of the training set were those qualified for final analysis after quality filtering (less than 5 % of the probes incorrectly interrogated (p-value <0.01). The validation set integrated 83 HCC patients treated with resection. The Institutional Review Boards of all participating centres approved the study.

Next Table 1 summarizes the clinical characteristics of the training cohort (n=221) and of the validation cohort (n=83)

Table 1. Clinical characteristics of HCC patients in the training and validation set

| Variable | Training | Validation |
|---|---|---|
| Median age | 66 | 66 |
| Gender (male) | 172 (78 %) | 68 (81 %) |
| Aetiology | | |
| - Hepatitic C<br>- Hepatitis B<br>- Alcohol<br>- Others | 101 (47 %)<br>44 (20 %)<br>35(16%)<br>37 (17 %) | 9 (11 %)<br>17(20%)<br>39 (47 %)<br>NA |
| Child-Pugh score:<br>- A<br>- B | <br>216(96%)<br>4(2%) | <br>74 (92 %)<br>9(8%) |
| Tumor size (cm)<br>- <2<br>- 2-3<br>- >3 | <br>26 (11 %)<br>73 (33 %)<br>122 (55 %) | <br>5(6%)<br>9 (11 %)<br>69 (83 %) |
| Multiple nodules<br>- Absent<br>- Present | <br>166 (75 %)<br>55 (25 %) | <br>69 (84 %)<br>16(16%) |
| Micro-vascular invasion<br>- Absent<br>- Present | <br>142 (64%)<br>77 (35 %) | <br>38 (46 %)<br>44 (54 %) |
| Satellites<br>- Absent<br>- Present | <br>158 (71 %)<br>63 (29 %) | <br>48 (58 %)<br>35 (42 %) |
| BCLC early stage (0-A) | 191 (87 %) | 59 (73 %) |
| Degree of tumor differentiation<br>- Well<br>- Moderately<br>- Poor | <br>34 (18 %)<br>105 (57 %)<br>45 (24 %) | <br>28 (34 %)<br>35 (43 %)<br>19(23%) |
| Bilirubin (> 1 mg/dl) | 92 (42 %) | 10 (19 %)(1) |
| Albumin (< 3.5 g/l) | 25 (11 %) | NA |
| Platelet count (<100000/$mm^3$) | 43 (19 %) | NA |

(continued)

| Variable | Training | Validation |
|---|---|---|
| AFP (> 100 mg/dl) | 51 (23 %) | 31 (41 %)(1) |
| Events<br>- Recurrence<br>- Death | <br>151 (69 %)<br>139 (62 %) | <br>NA<br>36 (43 %) |
| Median follow-up (months) | 48.5 | 35 |
| (1)Missing values, bilirubin (n=32) AFP (n=7); NA means not-available. AFP: alpha-fetoprotein. | | |

Data analysis:

To study differential methylation between HCC and normal liver tissues, probes (genomic sequences) containing single nucleotide polymorphisms (SNPs) or located on sex chromosomes were eliminated, leaving 434728 probes for analysis. Only those with a high signal quality (P<0.01) were considered. Probes hypomethylated (B<0.33) in at least 90 % of the normal liver samples and hypermethylated (B>0.5) in at least 5 % of the tumors (31052 CpG sites) and probes hypermethylated (B>0.5) in at least 90 % of the normal liver and hypomethylated (B<0.33) in at least 5 % of the tumors were selected (68086 CpG sites). The Beta value (B, β-value) is a value used to estimate the methylation level of the CpG locus using the ratio of intensities between methylated and unmethylated alleles. For prognosis purposes, analyses focused only on CpG islands located in gene regions TSS1500, TSS200, 5'UTR and 1stExon were filtered (n=84448), and which showed to be differentially methylated in tumors versus normal liver (n=11307), which allowed selecting methylation markers primarily de-regulated in cancer tissues.

In order to discover potential candidate HCC epidrivers, an F score directly proportional to intergroup variability (normal liver versus HCC) and inversely proportional to normal sample intragroup variability was calculated for all probes located in promoters and CpG islands. The first 500 ranked probes were grouped per gene. Genes with more than 5 hits were further studied as epidrivers. Previously reported epigenetically deregulated genes were also analysed, considering the mean of all probes located in the TSS200 region, which shows the best correlation with the expression. For those samples without TSS200 probes in the array, TSS1500 were used instead, as the closest marker available in the array.

A methylome signature (DNA methylation signature) was generated able to predict the risk of death for each individual measured by a mortality index (MI). The prediction signature was generated using the Random Survival Forest method (RSF) developed for variable selection (see Ishwaran et al., "Consistency of Random Survival Forests", Statistics and Probability Letters-2010, Vol. No.: 80, pp.: 1056-1064). RSF is a method for prediction and variable selection for right-censored survival and competing risk data by growing survival trees to estimate a cumulative hazard function (CHF), which derives from each tree of the RSF. Input variables were the filtered 11307 CpG sites. First, probes were randomly split in 6 sets of 2000 or less probes to fit a model each using the RSF method by growing 1000 survival trees. Variable importance scores (VIMP) were computed for all the probes used to grow the trees, and the 350 most informative from each model were selected to fit a new model using the same RSF method. Once the model was built, redundant and "noisy" variables were removed by generating incremental RSF models adding one more variable, ranked by the VIMP calculated in the previous model, at each step, getting an estimate of the error as a measure of how much misclassification increases, or decreases, for a new test case if a given variable was not available for that case. The final set of selected variables (genomic sequences/CpG sites defined by any of SEQ ID NO: 1 to 36, also called here 36 probes) was used to build the model having the lowest error rate using only the 2 most significant decimals of the obtained error rate. Thus, the methylation signature was based upon those 36 probes. The predictive accuracy of the 36 DNA-methylation signature was examined by bootstrap cross-validation.

From this last RSF model it was generated a DNA methylation based mortality index (i.e., Mortality Index (MI, range 36-360) for every individual computed as a sum of the CHF for each patient evaluated at a set of distinct time points weighed by the number of individuals at risk at different time points. High MI correlates with higher risk of death. Using this index, it was able to split the training set in 2 risk groups based on percentiles, selecting the patients with the 20 % highest levels [high risk (MI>230) and non-high risk (MI <230)] and test the variables selected as a prediction signature by confronting the high and low risk groups. To test the significance of the MI index, the same MI value used to split the training set was also used for the validation set. For the validation set, patients were split using the actual same value of MI generated in the training set.

Example 1. Methylation signature predicts survival in hepatocellular carcinoma

Random survival forests enabled to generate a methylation based signature (DNA methylation signature) that accurately discriminated patients based on their survival (Training set - Heptromic cohort, n=221). Table 1 (above) summarizes the clinical characteristics of this cohort, that mostly included males (172, 78%), with a median of 66 years/old, viral-related liver damage (HCV: 101,47%, HBV: 44, 20%), and a median tumor size of 3.5 cm. Patients had predominantly uninodular disease (166, 75%), and no microvascular invasion ( 142, 65 %) or satellites (158, 71 %) being the majority at early clinical stages (BCLC O/A: 191, 87%) with a median follow-up of 48.5 months (4 years). It was generated a methylation based signature formed by 36 unique "probes" (genomic regions of SEQ ID NOs: 1 to 36), which provided a DNA methylation-based mortality index (MI), ranging from 36-360. The mortality index (MI) is a metric based on the weighed hazard of death for an individual across all trees of RSF. This DNA methylation based mortality index significantly correlated with known clinical and pathological predictors of survival in surgically resected HCC such as satellites (P=0.02, FIG. 1C), multinodularity (P=0.002, FOG. 1 B), vascular invasion (P<0.001, FIG. 1A), Barcelona Clinic Liver Cancer (BCLC) staging (P=0.001, FIG. 1D), AFP (P=0.001, FIG. 1E), bilirubin (P=0.012, FIG. 1F ), platelet count (P=0.009, FIG. 1G) and albumin (P=0.03, FIG. 1H). Concordantly, univariate analysis showed that DNA methylation based mortality index significantly correlated with risk of death (p<0.0001) (FIG. 2A, wherein hazard ratio as a value of survival from 0-100 % of deaths is confronted with the MI). Median survival was significantly lower for patients with this DNA methylation based mortality index > 230 (which represent 20 % of the cohort) than for those with MI< 230 (13 vs 79.7 months respectively, p<0.001, FIG. 2B). Multivariate Cox regression modelling confirmed DNA methylation based mortality index as an independent predictor of survival (Hazard ratio (HR): 19.5, 95% CI 9.7-39.2, P<0.001) along with multinodularity (HR: 1.9, 95% CI 1.3-2.8, P<0.001) and male gender (HR: 0.53, 95% CI 0.35-0.79, P=0.002) in the training set. Similarly, DNA methylation based mortality index predicted tumor recurrence (R) (FIG. 2C) and Cox modelling showed it was an independent predictor of overall recurrence (HR: 5.8, 95% CI 3.1-11, P<0.001) along with multinodularity (HR: 1.8, 95% CI 1.1-3, P=0.007).

Outcome analyses were framed within current guidelines from the Progress Partnership for prognostic factor research (see Riley et al., "Prognosis Research Strategy (PROGRESS) 2: Prognostic Factor Research", PloS ONE-2013, Vol. No. 10, e1001380). Kaplan Meier plots and Cox regression were used to assess association with outcome of the methylation signature and clinical variables.

Prognostic performance of the DNA methylation based mortality index was validated in an independent dataset. The validation cohort also included HCC patients treated by surgical resection from different French institutions (n=83). Unlike the training set, the main etiology of these patients was related to alcohol intake (47 %, 39/83), and presented with more aggressive disease (microvascular invasion 54 %, satellites 42 % and tumors >3 cm in 83 %) and shorter median follow-up time (35 months). Patients with a high DNA methylation based mortality index (i.e. MI > 230; or splitable in group of high risk) had a significantly lower cancer-related survival (P=0.01, FIG. 3). Overall, these data indicate how epigenetic de-regulation correlates with patient outcome in surgically resected HCC and introduces a first-in-class DNA validated methylation based signature.

Similarly to other malignancies, there was a remarkable predominance of hypomethylated probes in HCC compared to normal liver (68 %). Hypomethylated probes were mainly located in the intergenic (39.9 %) and body regions (34.5 %), whereas hypermethylated probes were predominantly located in promoter areas (50.5 %). Regarding CpG island relation, hypomethylated probes were mainly located in open sea regions (63.55 %), while hypermethylated probes tended to locate in CpG islands (63.9 %) and shores (24.8 %).

Example 2. Integrated prognostic classification with transcriptome-based predictors.

In order to further characterize the subset of patients identified by DNA methylation signature, it was integrated whole-genome mRNA and methylation data of 205 HCCs.

RNA profiling was conducted using the Affymetrix® Human Genome U219 Array Plate, which is able to interrogate more than 20000 genes mapped. Processing of transcriptome data (i.e., normalization, background correction and filtering) was conducted as reported in Villanueva et al, Gastroenterolog-2011 (see *supra*). Prediction of liver cancer mRNA-based signatures in the training set was performed using the Nearest Template Prediction (NTP) method as implemented in the specific module of Gene Pattern software (see Hoshida et al., "Nearest template prediction: a single-sample-based flexible class prediction with confidence assessment" - PLoS ONE 2010;5(11):e15543). To provide biological insight on samples with high mortality risk score (MI >230, percentile 80), the *Gene Set Enrichment Analysis* (GSEA) was used. Gene ontology by PANTHER, INTERPRO and KEGG pathways enrichement analysis was performed using Database for Annotation, Visualization and Integrated Discovery (DAVID; v6.7).

Although data not shown, high (> 230) DNA methylation based mortality index was significantly enriched in tumors that harbored mRNA signatures capturing features related to progenitor cell origin such as Epithelial cell adhesion molecule (EpCAM, a diagnostic marker for various cancers) (p=0.009) and S2, a reporter molecular subclass of HCC characterized by cell proliferation and of potential progenitor cell origin (p=0.006). This was further confirmed when a *Gene Set Enrichment Analysis* (GSEA) was performed on those samples with the highest MI. In addition, it was found a significant enrichment of Gene Ontologies related to chromosomal dynamics and RNA processing in these samples (FDR<0.05). Conversely, tumors with lower MI were enriched in gene sets associated with metabolism and homeostasis.

Non-coding mRNA expression profile and DNA methylation data were tested in 205 samples. Patients with a high DNA-methylation-based mortality index had a differential non-coding mRNA expression pattern comprising 5 miRNAs and 1 small nucleolar RNA: miR-3193 ($p<0.001$), miR-27b-star ($p=0.002$), miR-422a ($p=0.02$), miR-378c ($p=0.03$), miR-938 ($p=0.03$) and SNORD126 (annotated as miT-1201 in the array) ($p=0.03$). All 6 non-coding RNAs were significantly down regulated in patients with high DNA-methylation-based mortality index.

All these exemplified data taken together allow concluding that the method of the invention, wherein DNA methylation type and levels (DNA methylation signature) is determined from at least the cytosines in the CpG sites defined by any of sequences SEQ ID NOs: 1 to 36, allows not only splitting patients with good and bad prognosis for the disease, but also characterizing a molecularly aggressive HCC subtype. The method accurately classifies HCC patients based on their outcome, and provides a quantitative death risk score (MI) to each subject that allows eliminating signal redundancy. This MI supposes a parameter making the method clinically applicable. Moreover, the DNA methylation signature used for the HCC prognosis according to the method of the invention is a biomarker independent of the cancer cause. In addition, the DNA methylation signature defined high risk of mortality in 20 % of patients in the cohort with best outcome (training set), compared to 50 % of patients in the validation cohort, and proved to have more aggressive tumors, thus allowing identification of patients with aggressive tumors across different stages.

Therefore, the method of the invention supposes a novel prognostic tool based on promoter DNA methylation changes in HCC that identifies subjects at high risk of death, at the same time it provides epigenetic characterization of the tumors. Fast detection of HCC tumors significantly enriched with mRNA subclasses capturing progenitor cell origin has in addition a major impact in the methods for deciding and/or recommending whether to initiate a medical regimen of a subject suffering HCC. In such a way, decisions and allocation of resources can be well-founded.

Also of note is the fact that the methods of the invention may be implemented using kits and processing data software commonly used in clinical practice. Thus, the proposed HCC prognosis method of the invention represents a real advantage and a real contribution to the art.

REFERENCES CITED IN THE APPLICATION

- Villanueva et al. "Combining Clinical, Pathology, and Gene Expression Data to Predict Recurrence of Hepatocellular Carcinoma", Gastroenterology-2011, Vol. No.140, pp.:1501-2.
- US2014004521
- US2012329672
- Hernandez-Vargas et al., "Hepatocellular Carcinoma Displays Distinct DNA Methylation Signatures with Potential as Clinical Predictors", PlosOne 2010 5(3) e9749.
- Song et al. "Elucidating the Landscape of Aberrant DNA Methylation in Hepatocellular Carcinoma", PlosOne 2013 8 e55761.
- Binkui et al, "CpG island Methylator Phenotype Associated with Tumor Recurrence in Tumor-Node-Metasyasis Stage I Hepatocellular Carcinoma", Annals of surgical ontology - 2010), Vol. No. 17(7), pp.:1917-26.
- Wu et al., "Predictive value of CpG island methylator phenotype for tumor recurrence in hepatitis B virus-associated hepatocellular carcinoma following liver transplantation", BMC cancer -2010, Vol. No. 10, pp.: 399.
- Cheng et al., "Correlation of CpG island methylator phenotype with poor prognosis in hepatocellular carcinoma", Experimental and Molecular Pathology-2010, Vol. No. 88(1), pp.: 112-117.
- Hoshida et al., "Nearest template prediction: a single-sample-based flexible class prediction with confidence assessment" - PLoS ONE 2010; Vol. No. 5(11):e15543.
- Riley et al., "Prognosis Research Strategy (PROGRESS) 2: Prognostic Factor Research", PIoS ONE-2013, Vol. No. 10, e1001380.
- Ishwaran H, Kogalur U, Blackstone E, Lauer M: Random survival forests. Ann Appl Stat 2008; 2: 841-860.
- Ishwaran et al., "Consistency of Random Survival Forests", Statistics and Probability Letters - 2010, Vol. No.: 80, pp.: 1056-1064.

## Claims

**1.** Method for the prognosis of hepatocellular carcinoma in an isolated sample of a subject, the method comprising providing a DNA methylation signature, defined by the qualitative and quantitative methylation analysis of a set of cytosines of CpG sites, said set comprising:

Cytosine at position 25062754 of human chromosome 20;
Cytosines at position 67571354, 30022699, 2932904 and 67313014 of human chromosome 16;
Cytosine at position 112194776 of human chromosome 6;
Cytosines at positions 147127012, 159943073 and 140660432 of human chromosome 3;
Cytosines at position 47797415, 26624823, 45236267 and 74648928 of human chromosome 2;
Cytosine at position 38747378 of human chromosome 19;
Cytosines at position 94928855 and 8559999 of human chromosome 8;
Cytosine at position 34443010 of human chromosome 21;
Cytosines at position 24899054, 23821445, and 69726620 of human chromosome 14;

Cytosine at position 107188416 of human chromosome 13;
Cytosine at position 48908469 of human chromosome 4;
Cytosines at position 108096745, 95401692, 155249746 and 5633086 of human chromosome 7;
Cytosines at position 75671378 and 97803058 of human chromosome 10;
Cytosine at position 83378614 of human chromosome 15;
Cytosines at position 24130633 and 32847789 of human chromosome 18;
Cytosine at position 114632040 of human chromosome 5;
Cytosines at position 66193820, 21279621 and 79197398 of human chromosome 17; and
Cytosine at position 35639656 of human chromosome 11;

being all cytosine positions in human chromosomes according to the chromosome map and sequence entries of database UCSC Genome Browser on Human February 2009, GRCh37/hg19 assembly of the University of California Santa Cruz (UCSC).

**2.** The method according to claim 1, wherein the set of cytosines of CpG sites consists in:

Cytosine at position 25062754 of human chromosome 20;
Cytosines at position 67571354, 30022699, 2932904 and 67313014 of human chromosome 16;
Cytosine at position 112194776 of human chromosome 6;
Cytosines at position 147127012, 159943073 and 140660432 of human chromosome 3;
Cytosines at position 47797415, 26624823, 45236267 and 74648928 of human chromosome 2;
Cytosine at position 38747378 of human chromosome 19;
Cytosines at position 94928855 and 8559999 of human chromosome 8;
Cytosine at position 34443010 of human chromosome 21;
Cytosines at position 24899054, 23821445, and 69726620 of human chromosome 14;
Cytosine at position 107188416 of human chromosome 13;
Cytosine at position 48908469 of human chromosome 4;
Cytosines at position 108096745, 95401692, 155249746 and 5633086 of human chromosome 7;
Cytosines at position 75671378 and 97803058 of human chromosome 10;
Cytosine at position 83378614 of human chromosome 15;
Cytosines at position 24130633 and 32847789 of human chromosome 18;
Cytosine at position 114632040 of human chromosome 5;
Cytosine at position 66193820, 21279621 and 79197398 of human chromosome 17; and
Cytosine at position 35639656 of human chromosome 11.

**3.** The method according to any of claims 1-2, wherein the DNA methylation signature is compared with a reference DNA methylation signature, and a bad prognosis is determined if the DNA methylation signature is within a range of DNA methylation signature reference indicative of bad prognosis.

**4.** The method according to any of claims 1-3, wherein the qualitative and quantitative methylation analysis comprises determining if in the isolated sample each cytosine of the set is methylated or not, and if methylated determining the level of methylation, expressed as a β-value ranging from 0 to 1, by means of formula (I):

$$\beta\text{-value}_{Cyt} = \max(y_{methCyt}, 0) / [\max(y_{unmethCyt}, 0) + \max(y_{methCyt}, 0)] \quad (I),$$

wherein max $y_{methCyt}$ is the maximal signal intensity detected for a methylated cytosine in the CpG site set; and max $y_{unmethCyt}$ is the maximal signal detected for an unmethylated cytosine in the CpG site set.

**5.** The method according to any of claims 1-4, wherein a DNA methylation based mortality index is obtained by means of the following steps:

(a) dropping down the DNA methylation signature from the isolated sample of a subject to a random survival forest (RSF) model for outcome prediction (prognosis), wherein said RSF model is built using Ntrees from the quantitative methylation analysis of a set of cytosines as defined in claim 1, said set of cytosines analysed in isolated samples of a training cohort consisting of subjects suffering from HCC;
(b) deriving a cumulative hazard function (CHF) from each of the trees of the model;

(c) determining an ensemble cumulative hazard function as an average of CHF of each tree; and
(d) obtaining a DNA methylation based mortality index as a weighted sum over the ensemble cumulative hazard function (CHF), weighted by the number of events at the different time points along analysis time of the training cohort.

**6.** The method according to any of claims 1-5, wherein the subject is a surgically resected subject.

**7.** The method according to any of claims 1-6, wherein the prognosis includes indicating the probability of recurrence of HCC, and/or the estimated overall survival, and/or the subtype of cancer, and/or the cancer-related survival.

**8.** The method according to claim 7, wherein the prognosis includes indicating the probability of recurrence of HCC.

**9.** The method according to claim 7, wherein the prognosis includes indicating the estimated overall survival.

**10.** The method according to claim 7, wherein the prognosis includes indicating classification of subtype of cancer.

**11.** The method according to any of claims 1-10, wherein the isolated biological sample is selected from the group consisting of a liver biopsy, blood, urine, and saliva.

**12.** The method according to any of claims 1-11, wherein analysis of CpG sites is performed with a set of DNA oligonucleotides that are suitable to detect sequences comprising SEQ ID NO: 1 to 36.

**13.** The method according to any of claims 1-12, further comprising determining in the isolated sample of the subject an RNA-transcription signature resulting from the qualitative and quantitative analysis of transcription of an RNA selected from the group consisting of miRNAs, mRNA and mixtures thereof.

**14.** A method of deciding and/or recommending whether to initiate a medical regimen of a subject suffering from HCC, which method comprises the steps of providing from an isolated sample of a subject a DNA methylation signature and determining the prognosis of hepatocellular carcinoma by means of a method as defined in any of claims 1-13; wherein if the subject is determined as having a bad prognosis, then a specific medical regimen comprising intensive monitoring after resection is recommended.

**15.** Use of means comprising DNA oligonucleotides suitable for determining DNA methylation signature of a set of CpG site cytosines, for the prognosis of hepatocellular carcinoma in any of the methods of claims 1-14.

(A)

(B)

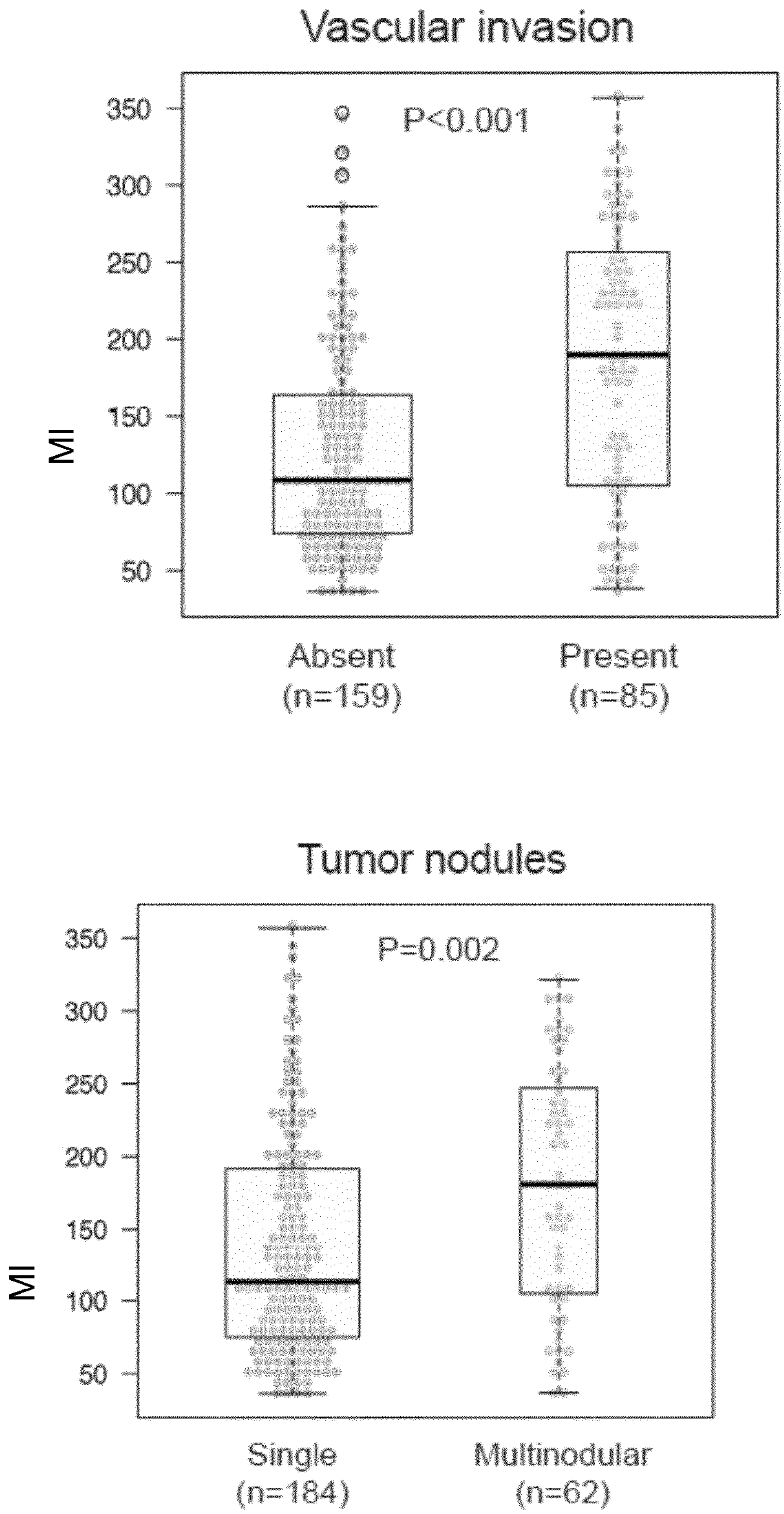
Vascular invasion
P<0.001
MI
350
300
250
200
150
100
50
Absent
(n=159)
Present
(n=85)
Tumor nodules
P=0.002
Single
(n=184)
Multinodular
(n=62)

FIG. 1

(C)

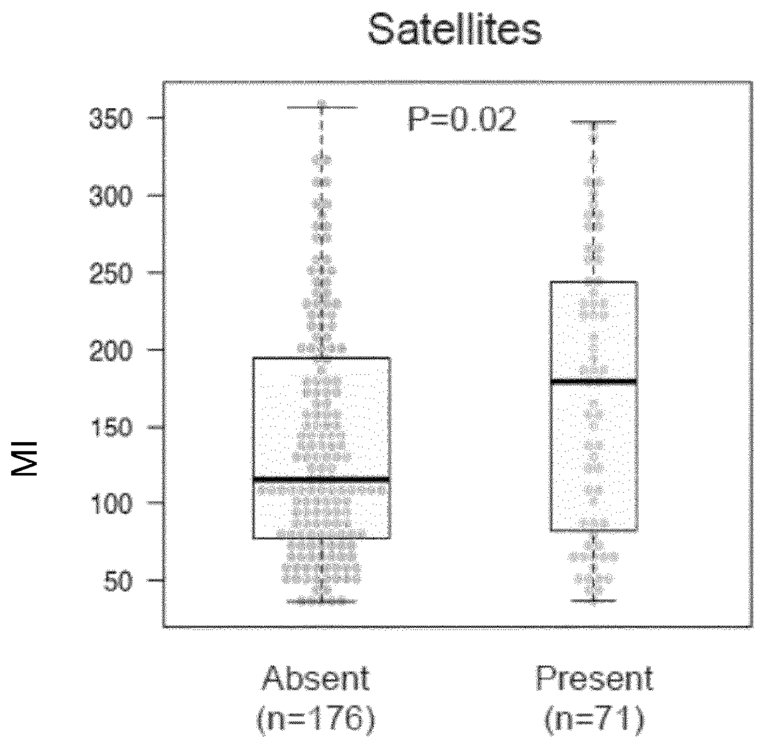
Satellites
P=0.02
350
300
250
200
150
100
50
MI
Absent
(n=176)
Present
(n=71)

(D)

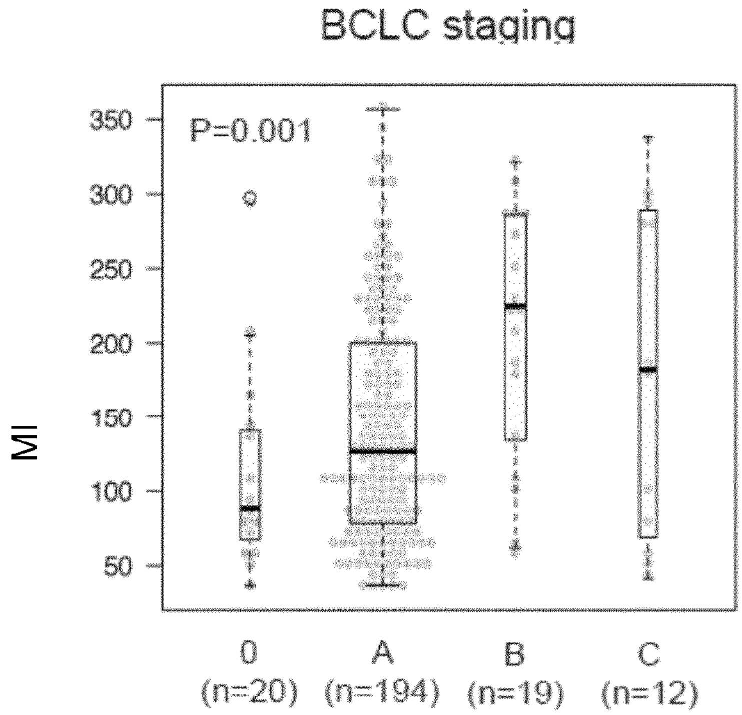
BCLC staging
P=0.001
350
300
250
200
150
100
50
MI
0
(n=20)
A
(n=194)
B
(n=19)
C
(n=12)

**Cont. FIG. 1**

(E)

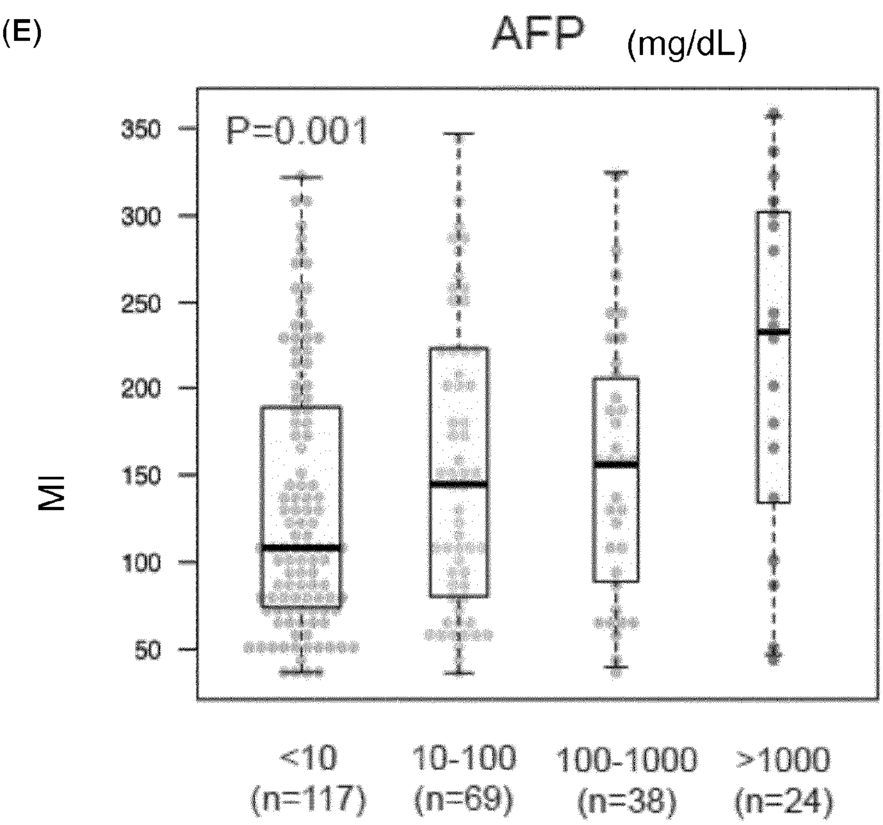
AFP (mg/dL)
P=0.001
MI
350
300
250
200
150
100
50
<10 (n=117)
10-100 (n=69)
100-1000 (n=38)
>1000 (n=24)

(F)

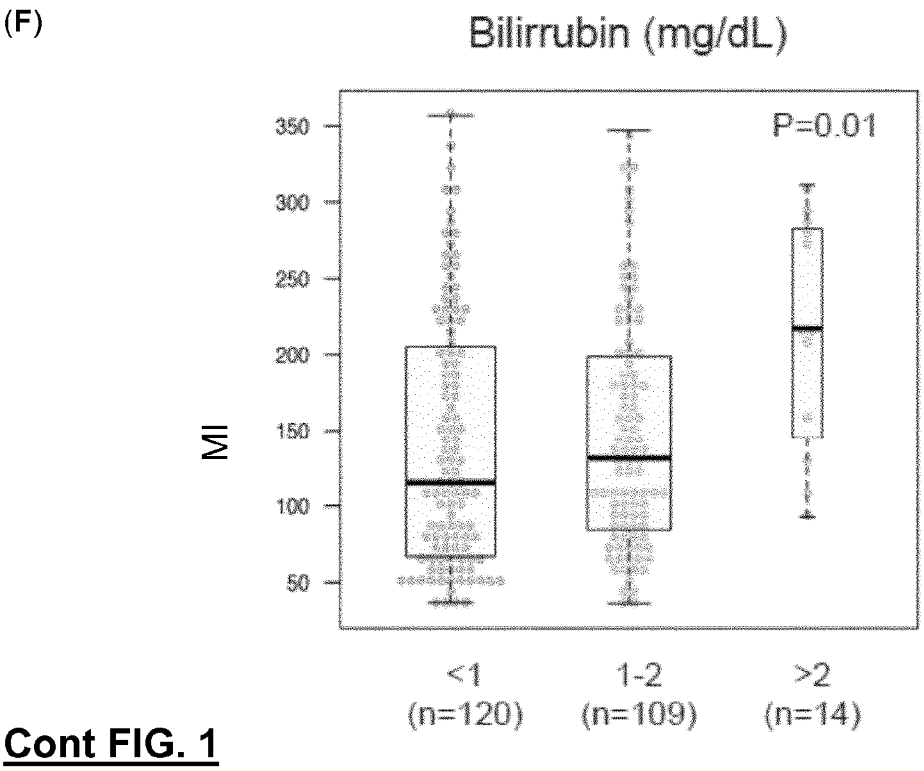
Bilirrubin (mg/dL)
P=0.01
MI
350
300
250
200
150
100
50
<1 (n=120)
1-2 (n=109)
>2 (n=14)

**Cont FIG. 1**

(G)

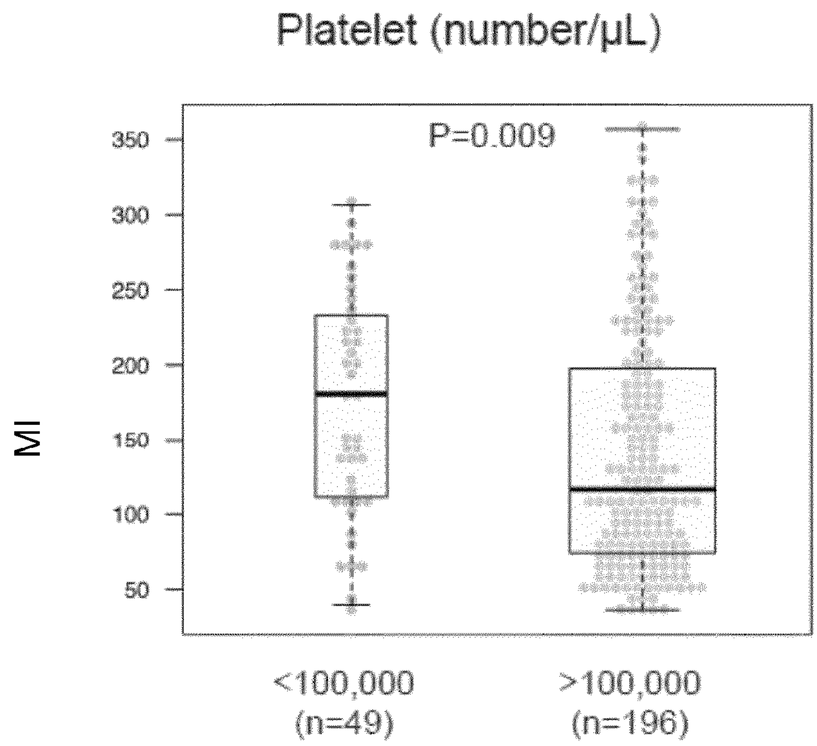
Platelet (number/µL)
P=0.009
MI
350
300
250
200
150
100
50
<100,000
(n=49)
>100,000
(n=196)

(H)

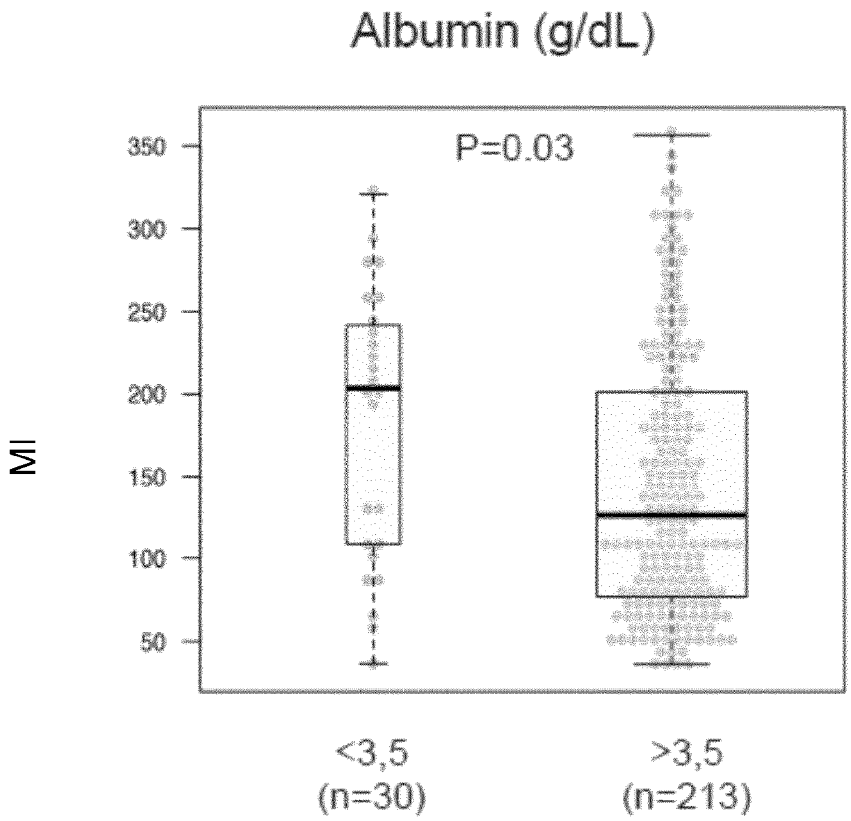
Albumin (g/dL)
P=0.03
MI
350
300
250
200
150
100
50
<3,5
(n=30)
>3,5
(n=213)

**Cont FIG. 1**

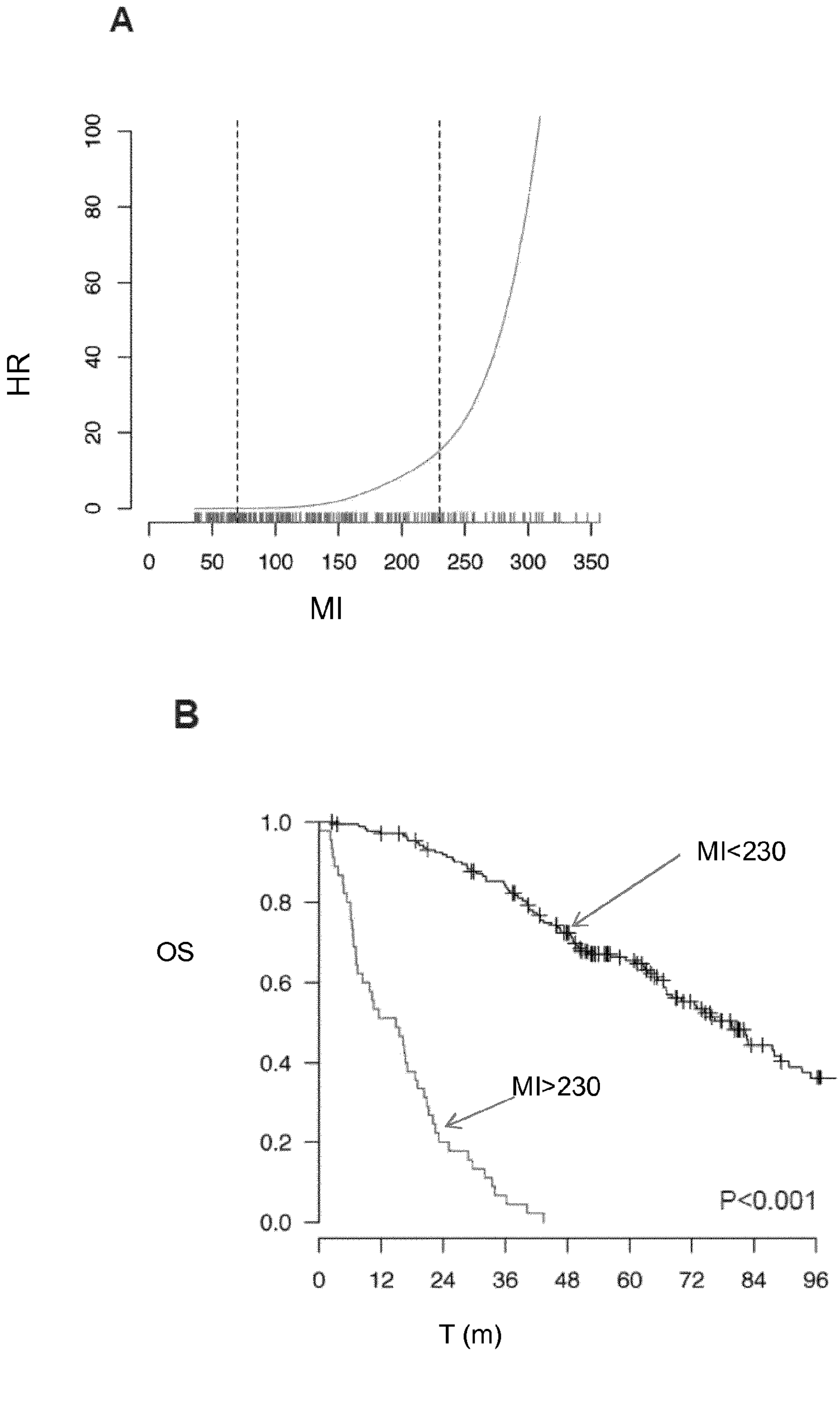
A
HR
100
80
60
40
20
0
0
50
100
150
200
250
300
350
MI
B
OS
1.0
0.8
0.6
0.4
0.2
0.0
MI<230
MI>230
P<0.001
0
12
24
36
48
60
72
84
96
T (m)

FIG. 2

C

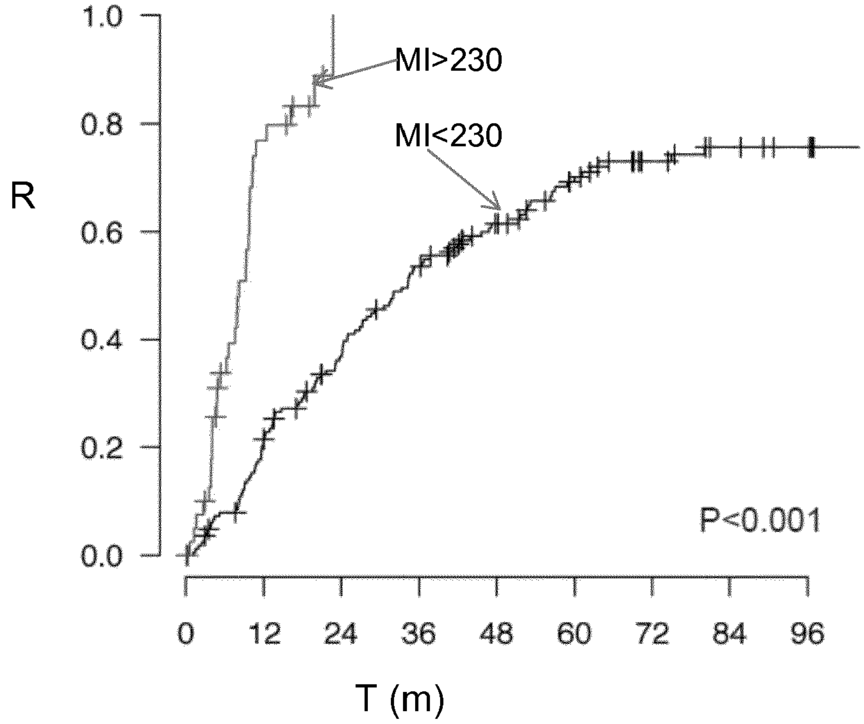


**Cont.FIG. 2**

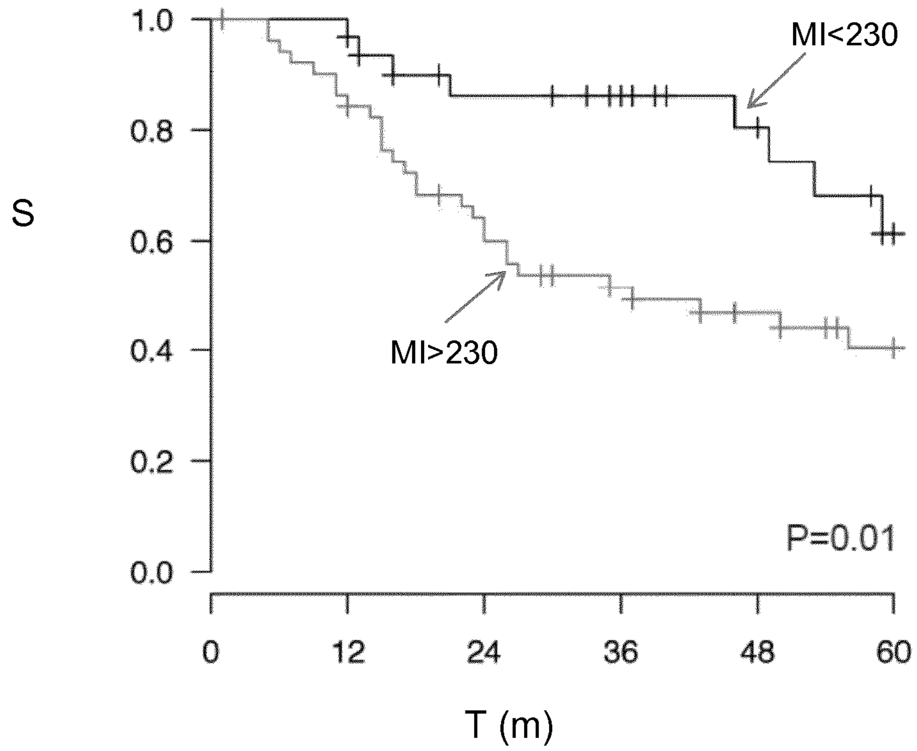


**FIG. 3**

Europäisches Patentamt
European Patent Office
Office européen des brevets

# EUROPEAN SEARCH REPORT

Application Number
EP 14 38 2535

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AUGUSTO VILLANEUVA ET AL: "Genome-wide methylation profiling identifies a 36-signature that predicts survival in surgically resected hepatocellular carcinoma", HEPATOLOGY, vol. 58, no. S1, 15 October 2013 (2013-10-15), page 1216A, XP055195223, ISSN: 0270-9139, DOI: 10.1002/hep.26883 * the whole document * ----- | 1-15 | INV. C12Q1/68 |
| A | WO 2008/107134 A2 (ROCHE DIAGNOSTICS GMBH [DE]; HOFFMANN LA ROCHE [CH]; MORIBE TOYOKI [JP) 12 September 2008 (2008-09-12) * claim 2; figure 5; examples 8, 9; tables 1, 2 * ----- | 1-15 | |
| A | WAY-CHAMP MAH ET AL: "Methylation Profiles Reveal Distinct Subgroup of Hepatocellular Carcinoma Patients with Poor Prognosis", PLOS ONE, vol. 9, no. 8, 5 August 2014 (2014-08-05), page e104158, XP055195311, DOI: 10.1371/journal.pone.0104158 * the whole document * ----- | 1-15 | TECHNICAL FIELDS SEARCHED (IPC) |
| | -/-- | | C12Q |

The present search report has been drawn up for all claims

1

| Place of search | Date of completion of the search | Examiner |
|---|---|---|
| The Hague | 12 June 2015 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 38 2535

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | CHENG Y ET AL: "Correlation of CpG island methylator phenotype with poor prognosis in hepatocellular carcinoma", EXPERIMENTAL AND MOLECULAR PATHOLOGY, ACADEMIC PRESS, US, vol. 88, no. 1, 1 February 2010 (2010-02-01), pages 112-117, XP026875014, ISSN: 0014-4800 [retrieved on 2009-10-29] * the whole document * ----- | 1-15 | |
| A,D | HECTOR HERNANDEZ-VARGAS ET AL: "Hepatocellular Carcinoma Displays Distinct DNA Methylation Signatures with Potential as Clinical Predictors", PLOS ONE, vol. 5, no. 3, 17 March 2010 (2010-03-17), page e9749, XP055163824, DOI: 10.1371/journal.pone.0009749 * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 12 June 2015 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 38 2535

This annex lists the patent family members relating to the patent documents cited in the above-mentioned European search report. The members are as contained in the European Patent Office EDP file on The European Patent Office is in no way liable for these particulars which are merely given for the purpose of information.

12-06-2015

| Patent document cited in search report | Publication date | Patent family member(s) | Publication date |
|---|---|---|---|
| WO 2008107134 A2 | 12-09-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014004521 A, Chao-Ting
- US 2012329672 A, Carlo M
- WO 2702007006 A


### Non-patent literature cited in the description


- **VILLANUEVA et al.** Combining Clinical, Pathology, and Gene Expression Data to Predict Recurrence of Hepatocellular Carcinoma. *Gastroenterolog,* 2011, vol. 140, 1501-2
- **HERNÁNDEZ-VARGAS et al.** Hepatocellular Carcinoma Displays Distinct DNA Methylation Signatures with Potential as Clinical Predictors. *PlosOne,* 2010, vol. 5 (3), e9749
- **SONG et al.** Elucidating the Landscape of Aberrant DNA Methylation in Hepatocellular Carcinoma. *PlosOne,* 2013, vol. 8, e55761
- **BINKUI et al.** CpG island Methylator Phenotype Associated with Tumor Recurrence in Tumor-Node-Metastasis Stage I Hepatocellular Carcinoma. *Annals of surgical oncology,* 2010, vol. 17 (7), 1917-26
- **WU et al.** Predictive value of CpG island methylator phenotype for tumor recurrence in hepatitis B virus-associated hepatocellular carcinoma following liver transplantation. *BMC cancer,* 2010, vol. 10, 399
- **CHENG et al.** Correlation of CpG island methylator phenotype with poor prognosis in hepatocellular carcinoma. *Experimental and Molecular Pathology,* 2010, vol. 88 (1), 112-117
- **ISHWARAN et al.** Consistency of Random Survival Forests. *Statistics and Probability Letters,* 2010, vol. 80, 1056-1064
- **ISHWARAN H ; KOGALUR U ; BLACKSTONE E ; LAUER M.** Random survival forests. *Ann Appl Stat,* 2008, vol. 2, 841-860
- **ISHWARAN et al.** Consistency of Random Survival Forests. *Statistics and Probability Letters-,* 2010, vol. 80, 1056-1064
- **RILEY et al.** Prognosis Research Strategy (PROGRESS) 2: Prognostic Factor Research. *PloS ONE-,* 2013, vol. 10, e1001380
- **VILLANUEVA et al.** *Gastroenterolog-,* 2011
- **HOSHIDA et al.** Nearest template prediction: a single-sample-based flexible class prediction with confidence assessment. *PLoS ONE,* 2010, vol. 5 (11), 15543
- **VILLANUEVA et al.** Combining Clinical, Pathology, and Gene Expression Data to Predict Recurrence of Hepatocellular Carcinoma. *Gastroenterology,* 2011, vol. 140, 1501-2
- **HERNANDEZ-VARGAS et al.** Hepatocellular Carcinoma Displays Distinct DNA Methylation Signatures with Potential as Clinical Predictors. *PlosOne,* 2010, vol. 5 (3), e9749
- CpG island Methylator Phenotype Associated with Tumor Recurrence in Tumor-Node-Metasyasis Stage I Hepatocellular Carcinoma. **BINKUI et al.** Annals of surgical ontology. 2010, vol. 17, 1917-26
- **HOSHIDA et al.** Nearest template prediction: a single-sample-based flexible class prediction with confidence assessment. *PLoS ONE,* 2010, vol. 5 (11), e15543
- **RILEY et al.** Prognosis Research Strategy (PROGRESS) 2: Prognostic Factor Research. *PloS ONE,* 2013, vol. 10, e1001380